# NOUVEAU FASCICULE DE BREVET EUROPEEN

(11) **EP 1 179 091 B2**
(45) Date de publication et mention de la décision concernant l'opposition: **25.02.2009**
(45) Mention de la délivrance du brevet: 13.10.2004
(21) Numéro de dépôt: 00929650.0
(22) Date de dépôt: 19.05.2000
(51) Int. Cl.: C12Q 1/68

(54) **PROCEDE D'ANALYSE DE LA PREDISPOSITION GENETIQUE D'UN PATIENT A AU MOINS UNE MALADIE ET AMPLIFICATION ADAPTEE A UN TEL PROCEDE**
ANALYSEVERFAHREN ZUR FESTSTELLUNG DER PRÄDISPOSITION ENES PATIENTEN AUF MINDESTENS EINE ERKRANKUNG SOWIE GEEIGNETE AMPLIFIZIERUNG FÜR EIN SOLCHES VERFAHREN
METHOD FOR ANALYSING A PATIENT'S GENETIC PREDISPOSITION TO AT LEAST A DISEASE AND AMPLIFICATION ADAPTED TO SUCH A METHOD

(30) Priorité: 20.05.1999 FR 9906599; 06.12.1999 FR 9915314
(43) Date de publication de la demande: 13.02.2002
(73) Titulaire: BIO MERIEUX, 69280 Marcy l'Etoile (FR)
(72) Inventeur: MOUGIN, Bruno, F-69003 Lyon (FR); TIERCY, Jean-Marie, F-12024 Chenes-Bougeries (FR)
(74) Mandataire: Guerre, Dominique
(86) Numéro de dépôt international: PCT/FR2000/001385
(87) Numéro de publication internationale: WO 2000/071750

(56) Documents cités:
- EP-A- 0 887 423
- WO-A-92/08117
- WO-A-97/20070
- WO-A-97/46700
- WO-A-98/35059
- WO-A-99/07883
- WO-A-99/19509
- FR-A- 2 679 252
- US-A- 5 468 611
- US-A- 5 567 809
- GARCIA-PACHECO J M ET AL: "Routine HLA DRB/DQB oligonucleotide typing by a non-radioactive dot-blot micromethod" JOURNAL OF IMMUNOLOGICAL METHODS,NL,ELSEVIER SCIENCE PUBLISHERS B.V.,AMSTERDAM, vol. 180, no. 1, 13 mars 1995 (1995-03-13), pages 35-43, XP004021068 ISSN: 0022-1759
- BEIN G ET AL.: "Rapid HLA-DRB1 genotyping by nested PCR amplification" TISSUE ANTIGENS, vol. 39, 1992, pages 68-73, XP000949259
- KIM H-Y ET AL.: "Predominance of HLA-DRB1*0405 in Korean patients with rheumatoid arthritis" ANNALS OF THE RHEUMATIC DISEASES, vol. 54, 1995, pages 988-990, XP000878854
- KAWAI S ET AL.: "A simple method of HLA-DRB typing using enzymatically amplified DNA and immobilized probes on microtiter plates" HUMAN IMMUNOLOGY, vol. 41, 1994, pages 121-126, XP000890112
- EVANS T I ET AL.: "The genotypic distribution of shared-epitope DRB1 alleles suggests a recessive mode of inheritance ot the rheumatoid arthritis disease-susceptibility gene" ARTHRITIS & RHEUMATISM, vol. 38, no. 12, 1995, pages 1754-1761, XP000878857
- DATABASE WPI , 1997 Derwent Publications Ltd., London, GB; AN 059711 XP002133035 "Detection and typing of class I MHC HLA-DR antigens - can check multiple specimens easily and type all HLA-DR (D-related) antigens known to be present in the Japanese population" -& JP 08 308596 A (WAKUNAGA SEIYAKU KK), 26 novembre 1997 (1997-11-26)
- ALLEN M ET AL.: "Allele-specific HLA-DRB1 amplification of forensic evidence samples with mixed genotypes" BIOTECHNIQUES, vol. 19, no. 3, 1995, pages 454-463, XP002133033
- DATABASE WPI , 1994 Derwent Publications Ltd., London, GB; AN 146988 XP002133036 "Oligo:nucleotide probes for HLA-DR typing of human DNA - and reagent kits containing probes, new amplification primers and buffers" -& JP 06 090757 A (KITASATO KENHYUSHO SH), 5 avril 1994 (1994-04-05)
- PETERSDORF E W ET AL: "POLYMORPHISM OF HLA-DRW52-ASSOCIATED DRB1 GENES AS DEFINED BY SEQUENCE-SPECIFIC OLIGONUCLEOTIDE PROBE HYBRIDIZATION AND SEQUENCING" TISSUE ANTIGENS,DK,MUNKSGAARD, COPENHAGEN, vol. 38, no. 4, 1 octobre 1991 (1991-10-01), pages 169-177, XP000673021 ISSN: 0001-2815
- HAWORTH S ET AL.: "Polymyalgia rheumatica is associated with both HLA-DRB1*0401 and DRB1*0404" BRITISH JOURNAL OF RHEUMATOLOGY, vol. 35, 1996, pages 632-635, XP000878853
- TAKEUCHI F ET AL.: "Positive and negative association of HLA-DR genotypes with Japanese theumatoid arthritis" CLINICAL AND EXPERIMENTAL RHEUMATOLOGY, vol. 14, 1996, pages 17-22, XP000878869
- NEPOM G T ET AL.: "HLA genes associated with rheumatoid arthritis" ARTHRITIS AND RHEUMATISM, vol. 32, no. 1, 1989, pages 15-21, XP000879219 cité dans la demande
- WAGNER U ET AL.: "HLA markers and prediction of clinical course and outcome in rheumatoid arthritis" ARTHRITIS & RHEUMATISM, vol. 40, no. 2, 1997, pages 341-351, XP000878856
- GAO X ET AL.: "DNA typing for class II HLA antigens with allele-specific or group-specific amplification" HUMAN IMMUNOLOGY, vol. 30, 1991, pages 147-154, XP000889911
- CEREB N ET AL: "LOCUS-SPECIFIC AMPLIFICATION OF HLA CLASS I GENES FROM GENOMIC DNA: LOCUS-SPECIFIC SEQUENCES IN THE FIRST AND THIRD INTRONS OF HLA-A, -B, AND -C ALLELES" TISSUE ANTIGENS,DK,MUNKSGAARD, COPENHAGEN, vol. 45, 1995, pages 1-11, XP000197333 ISSN: 0001-2815
- ANGELINI G ET AL.: "High-resolution analysis of the human HLA-DR polymorphism by hybridization with sequence-specific oligonucleotide probes" PROCEEDINGS OF THE NATIONAL ACADEMY OF SCIENCES USA, vol. 83, 1986, pages 4489-4493, XP002133034
- WORDSWORTH B P ET AL: "HLA-DR TYPING USING DNA AMPLIFICATION BY THE POLYMERASE CHAIN REACTION AND SEQUENTIAL HYBRIDIZATION TO SEQUENCE-SPECIFIC OLIGONUCLEOTIDE PROBES" IMMUNOGENETICS,DE,SPRINGER VERLAG, BERLIN, vol. 32, 1 janvier 1990 (1990-01-01), pages 413-418, XP002911048 ISSN: 0093-7711
- KIM ET AL: 'Predominance of HLA-DRB1*0405 in Korean patients with rheumatoid arthritis' ANNALS OF THE RHEUMATIC DISEASES vol. 54, 1995, SEOUL, KOREA, pages 988 - 990

## Description

La présente invention concerne un procédé d'analyse de la prédisposition génétique d'un patient à au moins la polyarthrite rhumatoïde.

Chaque individu dispose d'un patrimoine génétique propre, hérité de ses ascendants. Ce contexte génétique particulier peut parfois activement participer à l'apparition et/ou au développement de certaines affections : infections par un agent pathogène (virus du SIDA par exemple), maladies autoimmunes (maladies rhumatismales par exemple). Les gènes du Complexe Majeur d'Histocompatibilité (CMH) notamment les gènes codant pour les antigènes HLA (Human Leukocyte-Antigens), jouent un rôle prépondérant dans le développement des pathologies autoimmunes articulaires comme la polyarthrite rhumatoïde (Lawrence, 1970 ; Stastny, 1978 ; Khan, 1979 ; Stastny, 1983 ; Gregersen, 1986 ; Gregersen, 1987 ; Stastny, 1988 ; Todd, 1988 ; Wordsworth, 1989 ; Nepom, 1989 ; Hiraiwa, 1990 ; Nepom, 1991) ou la spondylarthrite ankylosante (Brewerton, 1973 ; Schlosstein, 1973 ; Benjamin, 1990).

Une partie importante de la composante génétique de la susceptibilité à la polyarthrite rhumatoïde a pu être associée aux gènes HLA-DRB, codant pour la chaîne β des molécules HLA-DR impliquées dans la présentation des peptides aux lymphocytes T, fonction pivot au coeur des mécanismes de régulation de la réponse immunitaire. Il a été montré plus précisément que la présence d'une séquence particulière de cinq acides aminés, correspondant aux positions 70 à 74 de la troisième région hypervariable des molécules HLA-DRβ1, était retrouvée pour différents allèles rapportés comme étant associés à la polyarthrite rhumatoïde. L'implication de cet « épitope partagé » correspondant aux séquences QKRAA ou QRRAA ou RRRAA (code à une lettre des acides aminés) est désormais bien documentée. Cette explication moléculaire est également cohérente avec l'observation d'une sévérité graduelle de la maladie lorsque le génotype comprend aucun, un ou deux allèles de susceptibilité, communément appelée effet dose.

La spondylarthrite ankylosante est une autre maladie inflammatoire, pour laquelle une très forte association avec l'antigène HLA-B27 (risque relatif: 69,1) est observée (Baarsma, 1992). En 1977, Schlosstein a publié la forte association entre HLA-B27 et différentes spondylarthropathies (Schlosstein, 1977). Différentes hypothèses sont avancées pour expliquer cette association, impliquant là encore la fonction de présentation de peptides spécifiques, des molécules HLA-B27.

Une association positive a été également publiée entre HLA-B27 et l'uvéite antérieure aiguë (risque relatif : 8,2).

La détection d'antigène(s) HLA-B27 présente un intérêt clinique certain, comme en atteste la pratique courante de cette analyse prescrite en consultation de rhumatologie.

La détection d'allèle(s) HLA-B*27 est également possible en utilisant les techniques de biologie moléculaire d'amplification et d'analyse des régions spécifiques d'intérêt.

Ainsi, en 1985, Weiss a publié l'organisation, la séquence et l'expression du gène HLA-B27. En 1991, Hill a publié une technique d'amplification par PCR des régions polymorphes du gène HLA-B et la détection du HLA-B*2703 par hybridation avec une sonde spécifique (Hill, 1991). En 1992, Dominguez a publié la description de la première méthode de génotypage du B27, après amplification par PCR des régions polymorphes du gène HLA-B.

*Actuellement en ce qui concerne la polyarthrite rhumatoïde, l'identification d'allèle(s) de susceptibilité est généralement faite en routine au cours d'un typage HLA-DR de faible résolution ou générique, permettant notamment la détection d'antigène(s) HLA-DR4 ou d'allèle(s) HLA-DRB1*gr04*.

Ces tests sont effectuées par les centres spécialisés dans l'étude des gènes HLA, tels les centres de transfusions sanguines ou certains hôpitaux spécialisés. Cette identification nécessite donc l'envoi d'un échantillon et l'utilisation d'une technologie assez lourde. Les conséquences négatives sont donc nombreuses, telles que :
- les risques de perte de l'échantillon,
- la longue durée de cette identification,
- le coût relativement élevé de ladite identification, et
- l'absence de contrôle par le demandeur sur le prestataire de services.

*L'état de la technique est très limité en ce qui concerne les associations HLA-DR et polyarthrite rhumatoïde, qui utilisent une technologie de biologie moléculaire.*

Dans le cadre de la spondylarthrite ankylosante, l'état de la technique est constitué essentiellement de techniques d'immunologie. C'est le cas de la demande de brevet WO-A-95/30152 qui permet l'identification de l'antigène B27. Ainsi, concernant la spondylarthrite ankylosante, une simple recherche d'antigène HLA-B27 est habituellement pratiquée en utilisant une technique sérologique (cytométrie de flux ou cytotoxicité, avec un anticorps spécifique anti-B27).

Ces techniques sont rapides mais des réactions faussement négatives sont parfois observées du fait du masquage des antigènes B27 par des autoanticorps ou des peptides (Neumüller, 1993 ; Kirveskari, 1997). De plus, comme toute technique d'analyse d'antigènes exprimés à la surface des lymphocytes, des précautions de bonne conservation des cellules sont nécessaires, parfois contraignantes.

Toujours dans ce domaine, l'état de la technique est également constitué de techniques de biologie moléculaire. Ainsi, le brevet US-A-4,971,902 concerne des sondes de diagnostic de la prédisposition d'un patient à la polyarthrite rhumatoïde. Ces sondes sont basées sur la reconnaissance du groupe d'allèles DRB1*gr04.

Toutefois, si ce groupe d'allèles DRB1*gr04 est effectivement associé à la polyarthrite rhumatoïde, tous les allèles actuellement connus (DRB1*gr0401 à DRB1*gr0427) ne sont pas forcément associés à cette maladie. Il peut en résulter que si l'on se limite à un typage du groupe d'allèles DRB1*gr04, en fonction des allèles présents, on pourra obtenir, outre de vrais positifs ou de vrais négatifs, des faux positifs qui vont alarmer inutilement le médecin et le patient.

*Qu'il s'agisse d'une technique sérologique (analyse des antigènes DR à l'aide d'une batterie de sérums de spécificité connue : résultats rendus selon la nomenclature DR1 à DR10) ou d'une technique de biologie moléculaire (analyse des allèles DRB1 : résultats rendus selon la nomenclature DRB1*01 à DRB1*10), le clinicien s'intéresse essentiellement à la présence d'antigène(s) DR4 ou d'allèle(s) DRB1*gr04, avec l'information « effet dose » éventuellement.*

*En cas de résultats suggérant une prédisposition à la maladie, un deuxième test est généralement pratiqué, utilisant une technique de biologie moléculaire dite de haute résolution dite de sous-typage du DR4, pour préciser l'allèle DRB1*gr04 (DRB1*gr0401 à gr0427 selon la nomenclature officielle en 1998), seuls les allèles DRB1*gr0401, gr0404, gr0405 et gr0408 étant rapportés comme étant associés à la maladie.*

Ces tests sont fiables mais longs, plusieurs jours, et coûteux.

Le procédé d'analyse revendiqué permet une analyse simplifiée sur le plan pratique, plus rapide, moins de deux heures, après préparation des amplicons, et peu onéreuse.

A cet effet, la présente invention concerne un procédé d'analyse de la prédisposition génétique d'un patient à la polyarthrite rhumatoïde, selon lequel :
on met un échantillon liquide contenant au moins un type d'amplicons, issus de l'amplification d'au moins une région polymorphe d'intérêt en rapport avec la polyarthrite rhumatoïde, en présence de sondes choisies de la façon suivante:
   - au moins une sonde à faible résolution capable de s'hybrider sur le groupe d'allèles DRB1*gr04,
   - au moins une sonde à haute résolution, associée à la susceptibilité génétique à la Polyarthrite Rhumatoïde, capable de s'hybrider avec les allèles suivants DRB1*0101, DRB1*gr0401, DRB1*gr0404, DRB1*gr0405, DRB1*gr0408 et DRB1*1402,
   - au moins une sonde à haute résolution, associée à la résistance génétique à la Polyarthrite Rhumatoïde, capable de s'hybrider avec les allèles suivants : DRB1*gr0402, DRB1*gr0403, DRB1*gr0406 et DRB1*gr0407,
   la ou les sondes à haute résolution permettant de discriminer le ou les allèles associés à la susceptibilité à la Polyarthrite rhumatoïde; et/ou le ou les allèles associés à la résistance à la Polyarthrite rhumatoïde, selon leur hybridation ou leur non hybridation.

Afin de détecter la présence d'un autre allèle, lorsqu'un seul allèle du gène ou du groupe d'allèles de ce gène a été détecté, par au moins une sonde spécifique de typage à faible résolution, correspondant au(x) autre(s) groupe(s) d'allèles, le procédé consiste à mettre les amplicons en présence d'au moins une sonde spécifique d'au moins un autre allèle, correspondant au polymorphisme dudit gène ou dudit groupe d'allèles de ce gène détecté par la ou les sondes à faible résolution.

Chaque sonde à faible ou à haute résolution, spécifique de la ou des maladies recherchées, comporte au moins un motif caractéristique de ladite ou desdites maladies recherchées.

De plus, on utilise au moins une sonde à faible résolution capable de s'hybrider sur le groupe d'allèles DRB1*01 et sur l'allèle DRB1*10.

Dans le cas où l'on souhaite détecter la présence d'un autre allèle lorsqu'un seul allèle du groupe d'allèles DRB1*gr04 a été détecté, on utilise au moins une sonde capable de s'hybrider sur les allèles suivants: DRB1*02,DRB1*03, DRB1*07, DRB1*08, DRB1*09, DRB1*11, DRB1*12, DRB1*13 et DRB1*14.

En ce qui concerne les sondes en rapport avec ces allèles, on utilise :
- une sonde SEQID NO 3 pour le typage de DRB1*gr04,
- deux sondes à haute résolution, associées à la susceptibilité génétique à la Polyarthrite Rhumatoïde :
   - SEQ ID NO 4 pour DRB1*gr0401,
   - SEQ ID NO7 pour DRB1*0101, DRB1*gr0404, DRB1*gr0405, et DRB1*gr0408, DRB1*1402,
- deux sondes à haute résolution, associées à la résistance génétique à la Polyarthrite Rhumatoïde :
   - SEQ ID NO 5 pour DRB1*gr0402, et
   - SEQ ID NO 6 pour DRB1*gr0403, DRB1*gr0406, DRB1*gr0407.

Plus précisément, on utilise également une sonde à haute résolution, SEQ ID NO 8 spécifique de DRB1*gr0405, associée à la susceptibilité génétique à la Polyarthrite Rhumatoïde.

De plus, on utilise les deux sondes suivantes pour le typage :
- SEQ ID NO 11 pour le groupe d'allèles DRB 1*01, et
- SEQ ID NO 15 pour l'allèle DRB1*10.

Dans le cas où l'on souhaite détecter la présence d'un autre allèle lorsqu'un seul allèle du groupe d'allèles DRB1*gr04 a été détecté, l'on utilise quatre sondes de typage suivantes :
- SEQ ID NO 13 pour DRB1*02,
- SEQ ID NO 14 pour DRB1*07, DRB1*09,
- SEQ ID NO 16 pour DRB1*08, DRB1*12, et
- SEQ ID NO 12 pour DRB1*03, DRB1*11, DRB1*13, DRB1*14.

Quel que soit le cas de figure, chaque sonde à faible ou à haute résolution, spécifique des allèles de susceptibilité génétique à la Polyarthrite Rhumatoïde et autres maladies associées, comporte l'un des motifs caractéristiques suivants : QKRAA, QRRAA ou RRRAA.

Au moins une sonde de contrôle, capable de s'hybrider avec l'ensemble des gènes DRB1, est utilisée pour permettre la détection de tous les gènes DRB1, tel que SEQ ID NO 1 : TTC GAC AGC GAC GTG GGG.

Si l'on veut en plus détecter des allèles de susceptibilité génétique à la Spondylarthrite Ankylosante et autres maladies associées, on utilise au moins une sonde à faible résolution, telle que SEQ ID NO 10, capable de s'hybrider sur le gène HLA-B, spécifique du groupe d'allèles HLA-B27.

Si l'on veut en plus détecter la susceptibilité génétique associée au Lupus Erythémateux Disséminé, à la Connectivite, au Syndrome de Sjögren et autres maladies associées, on utilise au moins une sonde à faible résolution, telle que SEQ ID NO 19, capable de s'hybrider sur le gène HLA-DR, spécifique du groupe d'allèles HLA-DRB1*03.

Quelle que soit la détection recherchée, au plus 38,89 % des bases d'une même sonde à faible résolution ou à haute résolution est remplacée par au moins une base analogue, telle que l'inosine.

Selon une variante de réalisation du procédé, chaque sonde spécifique à faible résolution ou à haute résolution, est placée dans un puits d'un plaque de microtitration indépendamment des autres sondes.

Selon une autre variante de réalisation du procédé, toutes les réactions sont réalisées simultanément.

Préalablement au procédé exposé ci-dessus, au moins une amplification de la ou des régions polymorphes d'intérêt est effectuée.

Une amplification de la ou des régions polymorphes d'intérêt associées au HLA-DR et une amplification de la ou des régions polymorphes d'intérêt associées au HLA-B sont effectuées simultanément.

Selon une variante de réalisation, on effectue une seconde amplification d'au moins une région plus ciblée que celle déjà effectuée sur la région polymorphe, cette région plus ciblée étant une région d'intérêt en rapport avec la polyarthrite rhumatoïde, et on met les amplicons obtenus en présence des sondes précédemment définies.

Selon cette variante, la première amplification est effectuée en même temps que ou préalablement à la seconde amplification plus ciblée.

La présente invention concerne également une amplification d'une séquence correspondant aux groupes d'allèles DRB1*gr04 en vue de son utilisation dans un procédé d'analyse de la prédisposition génétique d'un patient à la polyarthrite rhumatoïde, qui consiste à utiliser des amorces SEQ ID NO 20 en combinaison avec des amorces SEQ ID NO 21.

La présente invention concerne une amplification supplémentaire d'une séquence correspondant de l'allèle B27 en vue de son utilisation dans un procédé d'analyse de la prédisposition génétique d'un patient à la spondylarthrite ankylosante, qui consiste à utiliser des amorces SEQ ID NO 22, SEQ ID NO 23 et/ou SEQ ID NO 25 en combinaison avec des amorces SEQ ID NO 24 et/ou SEQ ID NO 26.

De plus, l'invention peut consister à combiner les deux amplifications décrites dans les deux paragraphes ci-dessus, en vue de leur utilisation dans un procédé. d'analyse de la prédisposition génétique d'un patient à la polyarthrite rhumatoïde et/éventuellement à la spondylarthrite ankylosante

Dans ce cas, l'amplification, dans laquelle la concentration finale en amorces permettant l'amplification de la séquence correspondant aux groupes d'allèles DRB1*gr04 est supérieure à la concentration finale en amorces permettant l'amplification de la séquence correspondant de l'allèle B27.

Dan une variante de réalisation, l'amplification précédente est associée à l'amplification d'une séquence correspondant à une région plus ciblée, incluse dans l'allèle DRB1*gr04, qui consiste à utiliser des amorces SEQ ID NO 21 en combinaison avec des amorces SEQ ID NO 27.

Les exemples ci-joint sont donnés à titre d'exemple explicatif et n'ont aucun caractère limitatif. Ils permettront de mieux comprendre l'invention.

Il s'agit d'un procédé dédié à l'analyse de prédispositions génétiques d'un individu à polyarthrite rhumatoïde, par une technique de biologie moléculaire permettant l'analyse simultanée de plusieurs gènes. Ce procédé peut être avantageusement appliqué à l'analyse des prédispositions génétiques d'un individu, pour cette maladie et éventuellement un ensemble de maladies apparentées, associée(s) à un ou plusieurs gènes. Ce procédé peut être appliqué à l'analyse des prédispositions génétiques d'un individu à la polyarthrite rhumatoïde et éventuellement à la spondylarthrite ankylosante.

L'intérêt de ce procédé est d'obtenir en une étape, avec un test multiple mais unique, un ensemble complet d'informations pertinentes d'intérêt clinique (diagnostique, pronostique et d'orientation thérapeutique). Le procédé se décompose en plusieurs étapes :
1) extraction d'acides nucléiques à partir d'un prélèvement biologique de l'individu,
2) amplification des régions d'intérêt, pour lesquelles un polymorphisme associé à une prédisposition génétique à une pathologie a été décrit, et
3) analyse simultanée des amplicons, utilisant un ensemble de réactions d'hybridation mettant en oeuvre un jeu de sondes moléculaires permettant l'analyse précise d'allèles ou de groupes d'allèles donnés.

### I - Exemple d'analyse simultanée des prédispositions génétiques d'un individu pour la polyarthrite rhumatoïde et la spondylarthrite ankylosante :

### 1°) Extraction d'acides désoxyribonucléiques (ADN) à partir d'un échantillon de sang-périphérique :

Cette extraction est réalisée de manière tout à fait classique. En fait, on peut utiliser n'importe quelle technique d'extraction d'ADN, permettant d'obtenir du matériel susceptible d'être ultérieurement amplifié par un procédé d'amplification comme la Polymerase Chain Reaction (PCR) Ces techniques de lyse des cellules, avec extraction puis purification des acides nucléiques sont habituellement celles recommandées pour des analyses génétiques, ou techniques rapides utilisant des produits commerciaux, telles que QIAmp Blood Kit (Marque déposée) de QIAGEN S.A.

### 2°) Amplification simultanée par PCR :

Cette amplification concerne les loci suivants
- le locus HLA-DR : incluant la région de l'exon 2 correspondant aux codons 5 à 94 selon la nomenclature officielle, des gènes HLA-DRB
- le locus HLA-B : incluant la région de l'exon 2 correspondant aux codons 25 à 114 selon la nomenclature officielle, du gène HLA-B

Le tableau 1 ci-dessous décrit les amorces utilisées lors de l'amplification des deux loci précédemment décrit. Il donne également l'ensemble des conditions physico-chimiques permettant la réalisation de cette amplification.

**Tableau 1 : Amplification simultanée des régions d'intérêt HLA-DR et HLA-B**

| | |
|---|---|
| Amorces | - P1 (amorce 5'-DR) : SEQ ID NO 20 |
| | CCG GAT CCT TCG TGT CCC CAC AGC ACG (5'>3') |
| | - P2 (amorce 3'-DR) : SEQ ID NO 21 |
| | TCG CCG CTG CAC TGT GAA G (5'>3') |
| | - P3 (amorce 5'-B) : SEQ ID NO 22 ou SEQ ID NO 23 |
| | GGG AGG AGC GAG GGG ACC CCA G (5'>3') ou |
| | GGG AGG AGC GAG GGG ACC GCA G (5'>3') |
| | - P4 (amorce 3'-B) : SEQ ID NO 24 |
| | ATC TCG GAC CCG GAG ACT (5'>3') |
| Mélange | - tampon 10X TEMAG (*) : 10 µl |
| réactionnel | - dNTPs (20 mM) : 1 µl (0,2 mM final) |
| | - P1 (30 µM) : 0,8 µl (0,25 µM final) |
| | - P2 (30 µM) : 0,8 µl (0,25 µM final) |
| | - P3 (30 µM) : 1,3 µl (0,4 µM final) |
| | - P4 (30 µM) : 1,3 µl (0,4 µM final) |
| | - AmpliTaq (5 U/µl) : 0.5 µl (2.5 U) |
| | - ADN : 100-500 ng |
| | - H₂O : QSP 100 µl |
| Programme | (5 min à 96°C) + 10 x (10 sec à 98°C + 30 sec à 65°C + 30 sec à |
| d'amplification | 72°C) + 30 x (20 sec à 96°C + 30 sec à 65°C + 30 sec à 72°C) |

| | |
|---|---|
| (*): Tampon 10X TEMAG : 500 mM Tris-HCl pH 8,8, 150 mM Sulfate Ammonium, 15 mM MgCl₂, 500 µM EDTA, 0,1 % gélatine | |

### 3°) Analyse simultanée des amplicons :

Cette analyse utilise un ensemble de réactions d'hybridation mettant en oeuvre un jeu de sondes oligonucléotidiques permettant l'analyse précise d'allèles ou de groupes d'allèles HLA-DRB1et HLA-B*27 importants pour l'étude de prédisposition génétique à la polyarthrite rhumatoïde et à la spondylarthrite ankylosante notamment.

Le tableau 2 décrit l'ensemble des sondes qui est utilisé pour la détection de ces deux maladies. Les indications qui sont données de la gauche vers la droite sont les suivantes :
- la référence de la sonde attribué dans la nomenclature HLA,
- le numéro de la séquence attribué dans ce document,
- le gène HLA concerné,
- la séquence constituant cette sonde, et
- la localisation des codons (trois nucléotides) sur les gènes HLA.

**Tableau 2 : Sondes oligonucléotidiques**

| Sonde | SEQ ID | Gène | Séquence (5'>3') | Localisation |
|---|---|---|---|---|
| | NO | HLA | | (codons) |
| C + | 1 | DR | TTC GAC AGC GAC GTG GGG | 40-45 |
| C - | 2 | - | TAT GAA ACT TAT GGG GAT AC | - |
| 4 | 3 | DR | GAT ACT TCT ATC ACC A | 29-34 |
| QK71 | 4 | DR | GAG CAG AAI CGG ICC | 69-73 |
| | | | *GAG CAG AAG CGG GCC* | |
| IDE71 | 5 | DR | CTG GAA GAC GAI CGG | 68-72 |
| | | | *CTG GAA GAC GAG CGG* | |
| E74 | 6 | DR | AGC AIA IGC IGG ICI AII | 69-75 |
| | | | *AGC AGA GGC GGG CCG AGG* | |
| QR71 | 7 | DR | CAG AGG CGI GII ICI GTG | 70-75 |
| | | | *CAG AGG CGG GCC GCG GTG* | |
| S57 | 8 | DR | GCC TAG CGC CGA GTA | 55-60 |
| C + (B) | 9 | B | AAA TAC CTC ATG GAG TGG GAG CC | 25-32 (*) |
| B27 | 10 | B | TGC CTT IGC CTT ICA GAT | 90-95 (*) |
| | | | *TGC CTT GGC CTT GCA GAT* | |
| 1 | 11 | DR | TGG CAG CTT AAG TTT GAA | 9-14 |
| 52 | 12 | DR | TAC TCT ACG TCT GAG T | 10-15 |
| 2 | 13 | DR | CAG CCT AAG AGG GAG TG | 10-15 |
| 7+9 | 14 | DR | IAG GTI GAC AIC GTG TGC | 74-79 |
| | | | *CAG GTG GAC ACC GTG TGC* | |
| 10 | 15 | DR | GGA GGA GGT TAA GTT | 8-13 |
| 8+12 | 16 | DR | CTC TAC GGG IGA GT | 10-15 |
| | | | *CTC TAC GGG TGA GT* | |
| 3 | 19 | DR | CCG GGT GGA CAA CIA C | 73-78 |
| | | | *CCG GGT GGA CAA CTA C* | |
| D1 | 17 | DR | GAA CAG CCA GAA GGA C | 61-66 |
| D6 | 18 | B | CTC GCT CTG GTT GTA GTA G | 106-113 (*) |

| | | | | |
|---|---|---|---|---|
| (*) sonde correspondant au brin complémentaire non-codant | | | | |

Pour certaines sondes, une deuxième séquence en caractères italiques précise la séquence naturelles, c'est-à-dire uniquement constituée des quatre nucléotides adénosine (A), Thymine (T), guanine (G) et cytosine (C). Les autres séquences reprennent les mêmes nucléotides à l'exception de la substitution de certains par des nucléotides différents. Dans le cas présent, il s'agit de l'inosine.

Certaines séquences ne contiennent pas d'inosine, c'est le cas des SEQ ID NO 1, 2, 3, 8, 9, 11, 12, 13, 15, 17 et 18. D'autres en contiennent peu, c'est le cas de la SEQ ID NO 19, où il y a une inosine sur un total de seize nucléotides, soit 6,25 % de différences par rapport à la séquence de base. D'autres en contiennent beaucoup plus, comme pour la SEQ ID NO 6, où il y a sept inosines sur un total de dix-huit nucléotides, soit environ 38,89 % de différences par rapport à la séquence de base.

L'utilisation des inosines permet d'améliorer encore la spécificité des sondes vis-à-vis des séquences auxquelles elles vont s'hybrider. La spécificité des sondes de captures est bien précisée dans le tableau 3 ci-après.

**Tableau 3 :**

| Spécificités principales des sondes de capture | | |
|---|---|---|
| Sonde | SEQ ID NO | Spécificité |
| C + | 1 | Tous les DRB1* |
| C - | 2 | Aucun |
| 4 | 3 | DRB1*gr04 |
| QK71 | 4 | DRB1*gr0401 notamment, allèle possédant le motif QKRAA correspondant à l'épitope partagé |
| IDE71 | 5 | DRB1*gr0402 notamment |
| E74 | 6 | DRB1*gr0403, gr0406 et gr0407 notamment |
| QR71 | 7 | DRB1*0101, gr0404, gr0405, gr0408 et 1402 notamment, allèle possédant le motif QRRAA correspondant à l'épitope partagé |
| S57 | 8 | DRB1*gr0405 notamment |
| C + (B) | 9 | Tous les B* |
| B27 | 10 | B*27 notamment |
| 1 | 11 | DRB1*01 |
| 52 | 12 | DRB1*03, 11, 13 et 14 notamment |
| 2 | 13 | DRB1*02 |
| 7+9 | 14 | DRB1*07 et 09 |
| 10 | 15 | DRB1*10 |
| 3 | 19 | DRB1*03 notamment |
| 8+12 | 16 | DRB1*08 et 12 notamment |

Dans ce tableau 3, le terme « notamment » est parfois associé à certains allèles. L'explication réside dans le fait qu'il existe d'autres allèles. Ainsi avec la SEQ ID NO 5, on détecte le DRB1*gr0402 mais également le DRB1*gr0414. Ce dernier est très rare, tellement rare qu'aucune étude n'a été entreprise à ce jour, pour lier sa présence à celle de la polyarthrite rhumatoïde. Néanmoins, les motifs de ces deux allèles étant semblables au niveau des régions polymorphes d'intérêt, il y a de forte chance que leur susceptibilité soit identique vis-à-vis de cette maladie.

Les sondes D1 et D6, correspondant aux SEQ ID NO 17 et 18 sont des sondes de détection.

Elles reconnaissent les régions observées chez tous les allèles d'un même gène. Ainsi, la sonde SEQ ID NO 17 est choisie dans une région conservée du gène HLA DR, alors que la sonde SEQ ID NO 18 est choisie dans une région conservée du gène HLA B.

Cette analyse des sondes peut être effectuée sur des plaques de micro-titration ou micro-plaques dont le format est normalisé. Ces plaques comportent des barrettes isolées de huit puits, qui peuvent être assemblées selon le nombre de tests à réaliser. Pour des impératifs de commodité et de coût, l'utilisation de deux barrettes par tests est avantageuse.

L'objectif est d'obtenir des résultats avec un minimum de barrettes, car le coût de fabrication doit être le plus réduit possible tout en ayant des prestations de très haut niveau. Ce minimum correspond à deux barrettes.

Dans le cas de la polyarthrite rhumatoïde et de la spondylarthrite ankylosante, on peut donc utiliser deux barrettes, qui seront respectivement appelées R1 et R2. Si pour la barrette R1, une possibilité est proposer, deux cas de figure sont possibles en ce qui concerne R2.

**Tableau 4 :**

| Organisation du multi-test (répartition des sondes dans les puits) | | | | | |
|---|---|---|---|---|---|
| Barrette R1 | | Barrette R2 | | Barrette R2bis | |
| C + | SEQ ID NO 1 | C + (B) | SEQ ID NO 9 | C + (B) | SEQ ID NO 9 |
| C - | SEQ ID NO 2 | B27 | SEQ ID NO 10 | B27 | SEQ ID NO 10 |
| 4 | SEQ ID NO 3 | 1 | SEQ ID NO 11 | 1 | SEQ ID NO 11 |
| QK71 | SEQ ID NO 4 | 52 | SEQ ID NO 12 | 52 | SEQ ID NO 12 |
| IDE71 | SEQ ID NO 5 | 2 | SEQ ID NO 13 | 2 | SEQ ID NO 13 |
| E74 | SEQ ID NO 6 | 7+9 | SEQ ID NO 14 | 3 | SEQ ID NO 19 |
| QR71 | SEQ ID NO 7 | 10 | SEQ ID NO 15 | 10 | SEQ ID NO 7 |
| S57 | SEQ ID NO 8 | 8+12 | SEQ ID NO 16 | 7+9+8+12 | SEQ ID NO 14 et 16 |

La barrette R1 comporte un contrôle positif (SEQ ID NO 1), qui permet de détecter tous les allèles du gène DRB1*, ce qui permet de contrôler que l'amplification a bien concerné la région d'intérêt comprise entre les amorces P1 et P2 décrites dans le tableau 1. Le contrôle négatif (SEQ ID NO 2) n'a pas d'objectif diagnostic, il n'est présent que pour répondre à certaines normes. Cette séquence n'est absolument pas spécifique du HLA, et correspond à une séquence aléatoire non retrouvée chez les gènes HLA.

La SEQ ID NO 3 permet le typage de l'ensemble des allèles qui constitue le groupe DRB1*gr04. Elle permet l'identification de tous les allèles DRB1*gr04, allèles appartenant au groupe défini par des techniques de typage HLA par sérologie, comme le groupe DR4. Il s'agit d'une sonde à faible résolution, c'est-à-dire que de nombreux allèles peuvent être reconnus par celle-ci.

Les SEQ ID NO 4 à 8 permettent, quant à elles, le sous-typage de certains des allèles qui constituent le groupe DRB1*gr04, en précisant le ou les allèles partageant une séquence particulière. Il s'agit de sondes à haute résolution, c'est-à-dire que quelques allèles, voire un seul, peuvent être reconnus par une de ces sondes.

Toutes ces sondes sont utilisées pour détecter les allèles possédant l'épitope partagé associés à la prédisposition génétique à la polyarthrite rhumatoïde. Plus particulièrement, les sondes SEQ ID NO 4 et 7 permettent de détecter les allèles associés à la susceptibilité à la polyarthrite rhumatoïde, , et les sondes SEQ ID NO 5 et 6 permettent de détecter les allèles associés à la résistance à ladite polyarthrite rhumatoïde. La sonde SEQ ID NO 8 permet de confirmer la présence d'un allèle DRB1*gr0405.

La barrette R2 comporte un contrôle positif (SEQ ID NO 9), qui permet de détecter les amplicons correspondant à l'exon 2 du gène HLA-B, ce qui permet de contrôler que l'amplification a bien concerné la région d'intérêt comprise entre les amorces P3 et P4 décrites dans le tableau 1.

Elle comporte d'autre part une SEQ ID 10, qui permet de détecter le groupe des allèles B*27, utilisée pour déterminer si un individu est prédisposé à développer une spondylarthrite ankylosante.

Pour le reste, cette barrette est constituée de sondes de faible résolution, à savoir les SEQ ID NO 11 à 16, qui permettent de compléter l'analyse des deux haplotypes de tout individu, notamment en indiquant si la présence d'allèles de susceptibilité ou de résistance à la polyarthrite rhumatoïde, révélée par la barrette R1, concerne l'un ou les deux haplotypes (effet dose).

Selon la balance entre allèle de susceptibilité, allèle de résistance et allèle neutre, les risques de développer une polyarthrite rhumatoïde plus ou moins sévère sont différents et les moyens thérapeutiques à mettre en oeuvre seront adaptés à ce diagnostic.

Dans le cas de la barrette 2bis, on reprend sensiblement la même configuration que la barrette R2, à l'exception de deux modifications.

Premièrement, deux réactions ont été regroupées. Ainsi, un seul puits contient en son sein deux sondes différentes, à savoir SEQ ID NO 14 et SEQ ID NO 16.

Le puits ainsi libéré permet l'ajout d'une nouvelle sonde SEQ ID NO 19 qui est associée à la présence d'allèles DRB1*03 (DR3). Il est en effet judicieux de pouvoir identifier de façon indépendante ce groupe d'allèles DRB1*03, retrouvé associé à d'autre maladies, comme le Lupus Erythémateux Disséminé, la Connectivite, le Syndrôme de Sjögren, le diabète insulino-dépendant par exemple.

### 4°) Préparation des réactifs permettant l'analyse des amplicons préparés :

Le principe d'hybridation inverse utilisé, est celui décrit dans le document WO-A-93/02213 de la demanderesse . Brièvement, les sondes spécifiques de capture sont adsorbées de façon passive sur le polystyrène des puits de barrettes montées en micro-plaques, grâce à une modification de leur extrémité 5' (présence d'une fonction -NH₂). Les sondes de détection sont des oligonucléotides couplés de façon covalente à l'enzyme HRP (Horse Radish Peroxydase) grâce à une modification de leur extrémité 5' (présence d'une fonction -NH₂). La composition du tampon d'hybridation a été modifiée comme suit :
- 75 mM Na₂HPO₄, 2H₂O,
- 25 mM NaH₂PO₄, H₂O,
- pH 6,8,
- 500 mM NaCl,
- 2 % PEG 4000,
- 0,65 % de Tween 20,
- 0,1 % de gélatine,
- 0,14 g/l d'ADN soniqué,
- 0,001 % de Ciprofloxacine chlorhydrate, et
- 0,02 % de Bromo-Nitro-Dioxane.

Un multi-test est constitué par une barrette R1 et une barrette R2 ou R2bis, comme décrit dans le tableau 4.

### 5°) Mode opératoire :

1) Dénaturation des amplicons : aux 100 µl de solution d'amplicons préparés, sont ajoutés 10 µl de réactif de dénaturation (2N NaOH). Incubation 5 minutes à 18-25°C.
2) Ajout de 2 ml de tampon d'hybridation, de 0,2 ml de solution contenant les sondes de détection.
3) Répartition du mélange, à raison de 100 µl dans chacun des seize puits sensibilisés correspondant à un multi-test. Couvrir d'une feuille autocollante.
4) Incubation 60 minutes à l'étuve à 37°C (35-39°C).
5) Elimination du matériel non hybridé par lavage à 18-25°C.
6) Révélation de la réaction enzymatique, en répartissant 100 µl par puits de solution de substrat (OPD, orthophénylène diamine). Incuber 20 minutes à 18-25°C, à l'obscurité.
7) Lecture des résultats : directe ou enregistrement des densités optiques (lecture à 492 nm).
8) Interprétation des résultats.

### II - Exemples et résultats:

Les résultats obtenus avec différents échantillons au typage HLA connu sont présentés ci-après :

### 1°) Premier échantillon :

Les deux tableaux 5 et 6 suivants représentent les résultats obtenus avec deux barrettes R1 et R2.

**Tableau 5 : Barrette R1**

| Barrette R1 | DO x 1000 | + / - |
|---|---|---|
| SEQ ID NO 1 | > 2500 | + |
| SEQ ID NO 2 | - | - |
| SEQ ID NO 3 | 898 | + |
| SEQ ID NO 4 | 256 | + |
| SEQ ID NO 5 | 1 | - |
| SEQ ID NO 6 | 0 | - |
| SEQ ID NO 7 | 10 | - |
| SEQ ID NO 8 | 9 | - |

**Tableau 6 : Barrette R2**

| Barrette R2 | DO x 1000 | + / - |
|---|---|---|
| SEQ ID NO 9 | >2500 | + |
| SEQ ID NO 10 | 0 | - |
| SEQ ID NO 11 | 0 | - |
| SEQ ID NO 12 | 0 | - |
| SEQ ID NO 13 | 2044 | + |
| SEQ ID NO 14 | 0 | - |
| SEQ ID NO 15 | 1 | - |
| SEQ ID NO 16 | 3 | - |

Du fait de l'utilisation d'une barrette R2, deux analyses sont possibles.

Premièrement, l'analyse du HLA-DR montre que :
- la sonde SEQ ID NO 1 est positive : l'amplification HLA-DR et le test d'hybridation ont correctement fonctionné,
- les sondes SEQ ID NO 3 et SEQ ID NO 4 sont positives : un allèle DRB1*gr0401 est présent, et
- la sonde SEQ ID NO 13 est positive : un allèle DRB1*02 est présent.

En conclusion, il y a présence d'un seul allèle de susceptibilité à la polyarthrite rhumatoïde (DRB1*gr0401), le second allèle étant DRB1*02 qui fait preuve de neutralité vis-à-vis de la polyarthrite rhumatoïde.

Deuxièmement, l'analyse du HLA-B montre que :
- la sonde SEQ ID NO 9 est positive : l'amplification HLA-B et le test d'hybridation ont correctement fonctionné, et
- la sonde SEQ ID NO 10 négative : il y a absence d'allèle B*27.

Le patient dont l'échantillon a été testé n'est pas susceptible à la spondylarthrite ankylosante.

Le typage HLA de ce premier échantillon était :
- HLA-DRB1*gr0401 / 1602, et
- HLA-B*5701.

### 2°) Deuxième échantillon :

Les deux tableaux 7 et 8 suivants représentent les résultats obtenus avec deux barrettes R1 et R2.

**Tableau 7 : Barrette R1**

| Barrette R1 | DO x 1000 | + / - |
|---|---|---|
| SEQ ID NO 1 | > 2500 | + |
| SEQ ID NO 2 | 0 | - |
| SEQ ID NO 3 | 261 | + |
| SEQ ID NO 4 | 0 | - |
| SEQ ID NO 5 | 312 | + |
| SEQ ID NO 6 | 0 | - |
| SEQ ID NO 7 | 4 | - |
| SEQ ID NO 8 | 0 | - |

**Tableau 8 : Barrette R2**

| Barrette R2 | DO x 1000 | + / - |
|---|---|---|
| SEQ ID NO 9 | >2500 | + |
| SEQ ID NO 10 | 0 | - |
| SEQ ID NO 11 | 0 | - |
| SEQ ID NO 12 | 0 | - |
| SEQ ID NO 13 | 2300 | - |
| SEQ ID NO 14 | 0 | - |
| SEQ ID NO 15 | 0 | - |
| SEQ ID NO 16 | 0 | - |

Il y a quatre sondes positives, qui sont :
- la sonde SEQ ID NO 1 : l'amplification HLA-DR et le test d'hybridation ont bien fonctionné,
- la sonde SEQ ID NO 3 : il y a présence d'au moins un allèle DRB1*gr04,
- la sonde SEQ ID NO 5 : il y a présence d'au moins un allèle DRB1*gr0402, et
- la sonde SEQ ID NO 9 : il y a présence d'au moins un allèle B* mais pas de B*27 puisque la SEQ ID NO 10 est négative.

Il s'agit donc présence d'un allèle DR4, mais d'un sous-type non impliqué dans la susceptibilité génétique à la PR (DRB1*gr0402). L'identification du deuxième allèle permet d'établir qu'il s'agit de DRB1*02.

Le typage HLA de ce troisième échantillon était :
- HLA-DRB1*gr0402 / 02, et
- HLA-B différent de B*27.

### 3°) Troisième échantillon :

Les deux tableaux 9 et 10 suivants représentent les résultats obtenus avec deux barrettes R1 et R2.

**Tableau 9 : Barrette R1**

| Barrette R1 | DO x 1000 | + / - |
|---|---|---|
| SEQ ID NO 1 | >2500 | + |
| SEQ ID NO 2 | - | - |
| SEQ ID NO 3 | 4 | - |
| SEQ ID NO 4 | 21 | - |
| SEQ ID NO 5 | 1145 | + |
| SEQ ID NO 6 | 0 | - |
| SEQ ID NO 7 | 8 | - |
| SEQ ID NO 8 | 0 | - |

**Tableau 10: Barrette R2**

| Barrette R2bis | DO x 1000 | + / - |
|---|---|---|
| SEQ ID NO 9 | >2500 | + |
| SEQ ID NO 10 | 2228 | + |
| SEQ ID NO 11 | 12 | - |
| SEQ ID NO 12 | 1278 | + |
| SEQ ID NO 13 | 877 | + |
| SEQ ID NO 14 | 0 | - |
| SEQ ID NO 15 | 0 | - |
| SEQ ID NO 16 | 5 | - |

Comme les deux exemples précédents, deux analyses sont possibles.

Premièrement, l'analyse du HLA-DR montre que :
- la sonde SEQ ID NO 1 est positive : l'amplification HLA-DR et le test d'hybridation ont correctement fonctionné,
- la sonde SEQ ID NO 3 est négative : il y a absence d'allèle DRB1*gr04,
- les sondes SEQ ID NO 5 et SEQ ID NO 12 sont positives : un allèle DRB1*11 ou 13 est présent, et
- la sonde SEQ ID NO 13 est positive : il y a présence d'un allèle DRB1*02.

En conclusion, il n'y a aucun allèle de susceptibilité à la polyarthrite rhumatoïde, les deux allèles DR ont cependant été identifiés au niveau générique.

Deuxièmement, l'analyse du HLA-B montre que :
- la sonde SEQ ID NO 9 est positive : l'amplification HLA-B et le test d'hybridation ont correctement fonctionné, et
- la sonde SEQ ID NO 10 est positive : il y a présence d'au moins un allèle B*27.

Le typage HLA de ce troisième échantillon était :
- HLA-DRB1*02 / 1301, et
- HLA-B*27.

### 4°) Quatrième échantillon :

Les deux tableaux 11 et 12 suivants représentent les résultats obtenus avec deux barrettes R1 et R2bis.

La configuration avec deux barrettes R1 et surtout R2bis (Voir tableau 4) permet, en plus de l'analyse de la susceptibilité génétique à la polyarthrite rhumatoïde et à la spondylarthrite ankylosante, de mieux analyser la susceptibilité génétique au Lupus Erythémateux Disséminé, aux Connectivités, au Syndrome de Sjögren et autres maladies rencontrées lors d'une consultation de rhumatologie. Ceci est réalisé en combinant dans un seul puits les SEQ ID NO 14 et 16, qui renferment les sondes 7, 8, 9 et 12, dont les spécificités sont décrites dans le tableau 3.

**Tableau 11 : Barrette R1**

| Barrette R1 | DO x 1000 | + / - |
|---|---|---|
| SEQ ID NO 1 | > 2500 | + |
| SEQ ID NO 2 | 0 | - |
| SEQ ID NO 3 | 275 | + |
| SEQ ID NO 4 | 241 | + |
| SEQ ID NO 5 | 0 | - |
| SEQ ID NO 6 | 0 | - |
| SEQ ID NO 7 | 0 | - |
| SEQ ID NO 8 | 0 | - |

**Tableau 12 : Barrette R2**

| Barrette R2 | DO x 1000 | + / - |
|---|---|---|
| SEQ ID NO 9 | >2500 | + |
| SEQ ID NO 10 | 0 | - |
| SEQ ID NO 11 | 0 | - |
| SEQ ID NO 12 | 1832 | + |
| SEQ ID NO 13 | 2 | - |
| SEQ ID NO 19 | 849 | + |
| SEQ ID NO 15 | 0 | - |
| SEQ ID NO 14 + 16 | 67 | - |

Il y a six sondes positives, qui sont :
- la sonde SEQ ID NO 1 : l'amplification HLA-DR et le test d'hybridation ont bien fonctionné,
- la sonde SEQ ID NO 3 : il y a présence d'au moins un allèle DRB1*gr04,
- la sonde SEQ ID NO 4 : il y a présence d'au moins un allèle DRB1*gr0401,
- la sonde SEQ ID NO 9 : il y a présence d'au moins un allèle B* mais pas de B*27 puisque la SEQ ID NO 10 est négative,
- la sonde SEQ ID NO 12 : il y a présence d'au moins un allèle DRB1*03, 11, 13 ou 14, et
- la sonde SEQ ID NO 19 : il y a présence d'au moins un allèle DRB1*03.

Il y a donc présence d'un allèle de susceptibilité génétique à la polyarthrite rhumatoïde du fait de l'existence de l'allèle DRB1*gr0401, et d'un allèle de susceptibilité génétique au Lupus Erythémateux Disséminé du fait de l'existence de l'allèle DRB1*03 chez ce patient. Il n'y a pas d'allèle concernant le B*27.

Le typage HLA de ce quatrième échantillon était :
- HLA-DRB1*gr0401 / 0301, et
- HLA-B différent de B*27.

### III - Optimisations des résultats :

L'objectif a été d'améliorer les conditions dans lesquelles doit être réalisée l'amplification. Les conditions optimales sont récapitulées dans le tableau 13 ci-dessous.

**Tableau 13 : Amplification simultanée et optimisée des régions d'intérêt HLA-DR et HLA-B**

| | |
|---|---|
| Amorces | - P1 (amorce 5'-DR) : SEQ ID NO 20 |
| | CCG GAT CCT TCG TGT CCC CAC AGC ACG (5'>3') |
| | - P2 (amorce 3'-DR) : SEQ ID NO 21 |
| | TCG CCG CTG CAC TGT GAA G (5'>3') |
| | - P3 (amorce 5'-B) : SEQ ID NO 25 |
| | GGG AGG AGC GAG GGG ACC GCA (5'>3') |
| | - P4 (amorce 3'-B) : SEQ ID NO 26 |
| | ATC TCG GAC CCG GAG ACT CG (5'>3') |
| Mélange | - tampon 10X TEMAG (*) : 10 µl |
| réactionnel | - dNTPs (20 mM) : 1 µl (0,2 mM final) |
| | - P1 (40 µM) : 1 µl (0,4 µM final) |
| | - P2 (40 µM) : 1 µl (0,4 µM final) |
| | - P3 (30 µM) : 1 µl (0,3 µM final) |
| | - P4 (30 µM) : 1 µl (0,3 µM final) |
| | - AmpliTaq (5 U/µl) : 0,5 µl (2,5 U) |
| | - ADN : 100-500 ng |
| | - H₂O : QSP 100 µl |
| Programme | (5 min à 96°C) + 10 x (10 sec à 98°C + 30 sec à 65°C + 30 sec à |
| d'amplification | 72°C) + 30 x (20 sec à 96°C + 30 sec à 62°C + 30 sec à 72°C) |

Les différences entre les conditions d'amplification du tableau 1 et du tableau 9 résident donc dans le choix des amorces P3 et P4 légèrement modifiées, de nouvelles concentrations en amorces P1, P2, P3 et P4 et une variation de température dans le programme d'amplification. L'apport dans les résultats de ces modifications est précisé dans les tableaux 14, 15 et 16 qui correspondent chacun à un échantillon différent de patients.

**Tableau 14 : Etude comparative du premier échantillon**

| | | | | Amorces et |
|---|---|---|---|---|
| | Conditions initiales | Amorces | Amplification | amplification |
| | | optimisées | optimisée | optimisées |
| SEQ ID NO 1 | > 2500 | > 2500 | > 2500 | > 2500 |
| SEQ ID NO 2 | 0 | 0 | 0 | 0 |
| SEQ ID NO 3 | 97 | 171 | 202 | 365 |
| SEQ ID NO 4 | 52 | 95 | 59 | 42 |
| SEQ ID NO 5 | 959 | 1552 | 966 | 1472 |
| SEQ ID NO 6 | 0 | 0 | 0 | 0 |
| SEQ ID NO 7 | 17 | 44 | 31 | 45 |
| SEQ ID NO 8 | 0 | 16 | 0 | 3 |
| SEQ ID NO 9 | 1237 | 1708 | 1627 | 1792 |
| SEQ ID NO 10 | 211 | 363 | 394 | 414 |
| SEQ ID NO 11 | 3 | 0 | 0 | 0 |
| SEQ ID NO 12 | 882 | 895 | 737 | 791 |
| SEQ ID NO 13 | 0 | 0 | 0 | 0 |
| SEQ ID NO 14 | 0 | 0 | 0 | 0 |
| SEQ ID NO 15 | 0 | 0 | 0 | 0 |
| SEQ ID NO 16 | 0 | 0 | 0 | 0 |

Le typage de ce premier échantillon est de DRB1*gr0404 / 52.

**Tableau 15 : Etude comparative du deuxième échantillon**

| | | | | Amorces et |
|---|---|---|---|---|
| | Conditions initiales | Amorces | Amplification | amplification |
| | | optimisées | optimisée | optimisées |
| SEQ ID NO 1 | 789 | 1335 | 1240 | 1914 |
| SEQ ID NO 2 | 0 | 0 | 0 | 0 |
| SEQ ID NO 3 | 353 | 649 | 635 | 944 |
| SEQ ID NO 4 | 14 | 49 | 45 | 102 |
| SEQ ID NO 5 | 104 | 232 | 228 | 463 |
| SEQ ID NO 6 | 0 | 0 | 0 | 0 |
| SEQ ID NO 7 | 0 | 0 | 0 | 0 |
| SEQ ID NO 8 | 0 | 0 | 0 | 0 |
| SEQ ID NO 9 | 1822 | > 2500 | 2120 | 2240 |
| SEQ ID NO 10 | 0 | 0 | 0 | 0 |
| SEQ ID NO 11 | 0 | 0 | 0 | 0 |
| SEQ ID NO 12 | 0 | 0 | 0 | 0 |
| SEQ ID NO 13 | 1 | 5 | 2 | 1 |
| SEQ ID NO 14 | 0 | 0 | 0 | 0 |
| SEQ ID NO 15 | 0 | 0 | 0 | 0 |
| SEQ ID NO 16 | 0 | 0 | 3 | 0 |

Le typage de ce deuxième échantillon est de DRB1*gr0401 / gr0402.

**Tableau 16 : Etude comparative du troisième échantillon**

| | | | | Amorces et |
|---|---|---|---|---|
| | Conditions initiales | Amorces | Amplification | amplification |
| | | optimisées | optimisée | optimisées |
| SEQ ID NO 1 | 615 | 1156 | 1052 | 2098 |
| SEQ ID NO 2 | 0 | 0 | 0 | 0 |
| SEQ ID NO 3 | 429 | 691 | 679 | 1381 |
| SEQ ID NO 4 | 0 | 0 | 0 | 0 |
| SEQ ID NO 5 | 0 | 0 | 0 | 0 |
| SEQ ID NO 6 | 8 | 23 | 17 | 66 |
| SEQ ID NO 7 | 14 | 26 | 21 | 66 |
| SEQ ID NO 8 | 64 | 174 | 131 | 405 |
| SEQ ID NO 9 | 1348 | 2377 | 2089 | 2191 |
| SEQ ID NO 10 | 296 | 622 | 567 | 490 |
| SEQ ID NO 11 | 0 | 0 | 0 | 0 |
| SEQ ID NO 12 | 0 | 0 | 0 | 0 |
| SEQ ID NO 13 | 3 | 0 | 1 | 0 |
| SEQ ID NO 14 | 0 | 0 | 0 | 0 |
| SEQ ID NO 15 | 0 | 0 | 0 | 0 |
| SEQ ID NO 16 | 0 | 2 | 0 | 0 |

Le typage de ce troisième échantillon est de DRB1*gr0403 / gr0405.

Toutes ces valeurs sont des valeurs de DO multipliées par mille (DO x 1000). La valeur maximale lisible étant de 2,5, lorsque le chiffre est supérieur à cette valeur, on indique uniquement que la valeur est supérieure à 2500 (>2500).

On remarque que toutes les valeurs significatives les plus élevées ont été mises en exergue par épaississement du trait. Il s'agit uniquement des sondes positives. Pour le premier échantillon, sur les sept valeurs significatives, toutes sont issues des conditions optimisées par rapport aux conditions initiales. Trois sont associées à l'optimisation des amorces et quatre à l'optimisation des amorces et de l'amplification. Pour le deuxième échantillon, sur les cinq valeurs significatives, toutes sont issues des conditions optimisées par rapport aux conditions initiales. Une est associée à l'optimisation des amorces et quatre à l'optimisation des amorces et de l'amplification. Pour le troisième échantillon, sur les sept valeurs significatives, toutes sont issues des conditions optimisées par rapport aux conditions initiales. Deux sont associées à l'optimisation des amorces et cinq à l'optimisation des amorces et de l'amplification.

L'optimisation de l'amplification seule se justifie moins que l'optimisation des amorces seule ou l'optimisation des amorces et de l'amplification. Néanmoins on remarque que sur les séquences correspondant aux dix-neuf valeurs significatives, dix-huit sont en faveur de l'optimisation de l'amplification par rapport aux conditions initiales.

En comparant les techniques d'amplification selon le tableau 1 et selon le tableau 13, on s'aperçoit que l'amplification est plus efficace lorsque la concentration finale en amorces permettant l'amplification de la séquence correspondant aux groupes d'allèles DRB1*gr04 est supérieure à la concentration finale en amorces permettant l'amplification de la séquence correspondant à l'allèle B27.

### IV - Perfectionnements :

Dans certains cas des ambiguïtés peuvent apparaître. Ainsi les amorces n'étant pas spécifiques du DR4, d'autres allèles sont amplifiés ce qui peut entraîner des difficultés d'interprétation. C'est par exemple le cas dans les trois exemples ci-dessous, la seule solution est alors d'effectuer une amplification plus ciblée sur le DR4, à l'aide d'amorces particulières telles que décrites dans le tableau 17 ci-dessous :

**Tableau 17 : Amplification ciblée d'une région d'intérêt HLA-DR**

| | |
|---|---|
| Amorces | - P5 (amorce 5'-DR) : SEQ ID NO 27 |
| | GTT TCT TGG AGC AGG TTA AAC (5'>3') |
| | - P2 (amorce 3'-DR) : SEQ ID NO 21 |
| | TCG CCG CTG CAC TGT GAA G (5'>3') |
| Mélange | - tampon Tris-HCl pH 8,3 : 50 µM |
| réactionnel | - dNTPs (20 mM) : 1 µl (0,2 mM final) |
| | - P2 (30 µM) : 1 µl (0,3 µM final) |
| | - P5 (30 µM) : 1 µl (0,3 µM final) |
| | - AmpliTaq (5 U/)µl) : 0,3 µl (1,5 U) |
| | - ADN : 100-500 ng |
| | - H₂O : QSP 100 µl |
| Programme | (2 min à 95°C) + 32 x (30 sec à 95°C + 30 sec à 55°C + 30 sec à |
| d'amplification | 72°C) + 30 x (20 sec à 96°C + 30 sec à 62°C + 30 sec à 72°C+7 min |
| | à 72°C) |

Les exemples qui montrent de telles ambiguïtés et leur résolution sont les suivants.

### 1°) Premier échantillon :

**Tableau 18 : Barrette R1**

| Barrette R1 | DO x 1000 | + / - |
|---|---|---|
| SEQ ID NO 1 | >2500 | + |
| SEQ ID NO 2 | < 50 | - |
| SEQ ID NO 3 | 197 | + |
| SEQ ID NO 4 | 182 | + |
| SEQ ID NO 5 | 1692 | + |
| SEQ ID NO 6 | 0 | - |
| SEQ ID NO 7 | 11 | - |
| SEQ ID NO 8 | 3 | - |

**Tableau 19 : Barrette R2**

| Barrette R2bis | DO x 1000 | + / - |
|---|---|---|
| SEQ ID NO 9 | >2500 | + |
| SEQ ID NO 10 | 0 | - |
| SEQ ID NO 11 | 2 | - |
| SEQ ID NO 12 | 1519 | + |
| SEQ ID NO 13 | 0 | - |
| SEQ ID NO 14 | 0 | - |
| SEQ ID NO 15 | 0 | - |
| SEQ ID NO 16 | 0 | - |

Dans ce cas, il n'est pas possible, sans prendre de risque de différencier dans le groupe DRB1*gr04 (SEQ ID NO 3 est positive) entre DRB1*gr0401 (SEQ ID NO 4 est positive) et DRB1*gr0402 (SEQ ID NO 5 est positive). L'autre allèle est un gr52 (SEQ ID NO 12 est positive). De plus, les chiffres bruts tendent à privilégier DRB1*gr0402 puisque la SEQ ID NO 5 a une valeur positive de 1692, alors que pour DRB1*gr0401 la SEQ ID NO 4 n'est positive qu'avec une valeur de 182.

Pourtant la seconde analyse va permettre de différencier ces deux allèles. C'est ce qui est bien représenté sur les tableaux 20 et 21.

**Tableau 20 : Barrette R1**

| Barrette R1 | DO x 1000 | + / - |
|---|---|---|
| SEQ ID NO 1 | 2181 | + |
| SEQ ID NO 2 | < 50 | - |
| SEQ ID NO 3 | 1281 | + |
| SEQ ID NO 4 | 200 | + |
| SEQ ID NO 5 | 0 | - |
| SEQ ID NO 6 | 0 | - |
| SEQ ID NO 7 | 1 | - |
| SEQ ID NO 8 | 0 | - |

**Tableau 21 : Barrette R2**

| Barrette R2bis | DO x 1000 | + / - |
|---|---|---|
| SEQ ID NO 9 | ND | ? |
| SEQ ID NO 10 | ND | ? |
| SEQ ID NO 11 | 0 | - |
| SEQ ID NO 12 | 0 | - |
| SEQ ID NO 13 | 0 | - |
| SEQ ID NO 14 | 0 | - |
| SEQ ID NO 15 | 0 | - |
| SEQ ID NO 16 | 0 | - |

On voit donc bien sur cette seconde analyse qu'il s'agit de DRB1*gr0401 (SEQ ID NO 4 est positive), alors que DRB1*gr0402 (SEQ ID NO 5) est négative . La différenciation entre DRB1*gr0401 et DRB1*gr0402 est donc possible, ce qui a une grande incidence puisque ces deux allèles n'ont pas le même impact sur la polyarthrite rhumatoïde. Ainsi, DRB1*gr0401 est associé à la susceptibilité génétique à la polyarthrite rhumatoïde, et DRB1*gr0402 est associé à la résistance génétique à la polyarthrite rhumatoïde.

Le typage de ce premier échantillon est donc DRB1*gr0401 / gr52.

### 2°) Deuxième échantillon :

**Tableau 22 : Barrette R1**

| Barrette R1 | DO x 1000 | + / - |
|---|---|---|
| SEQ ID NO 1 | >2500 | + |
| SEQ ID NO 2 | <50 | - |
| SEQ ID NO 3 | 193 | + |
| SEQ ID NO 4 | 21 | - |
| SEQ ID NO 5 | 0 | - |
| SEQ ID NO 6 | 0 | - |
| SEQ ID NO 7 | 72 | + |
| SEQ ID NO 8 | 137 | + |

**Tableau 23 : Barrette R2**

| Barrette R2bis | DO x 1000 | + / - |
|---|---|---|
| SEQ ID NO 9 | >2500 | + |
| SEQ ID NO 10 | 1 | - |
| SEQ ID NO 11 | 1 | - |
| SEQ ID NO 12 | 1827 | + |
| SEQ ID NO 13 | 0 | - |
| SEQ ID NO 14 | 0 | - |
| SEQ ID NO 15 | 0 | - |
| SEQ ID NO 16 | 3 | - |

Dans ce cas, il n'est pas possible, sans prendre de risque de différencier dans le groupe DRB1*gr04 (SEQ ID NO 3 est positive) entre DRB1*gr0404 (SEQ ID NO 7 est positive) et DRB1*gr0405 (SEQ ID NO 7 et SEQ ID NO 8 sont toutes les deux positives). L'autre allèle est un gr52 (SEQ ID NO 12 est positive).

Pourtant la seconde analyse va permettre de différencier ces deux allèles. C'est ce qui est bien représenté sur les tableaux 24 et 25.

**Tableau 24 : Barrette R1**

| Barrette R1 | DO x 1000 | + / - |
|---|---|---|
| SEQ ID NO 1 | >2500 | + |
| SEQ ID NO 2 | < 50 | - |
| SEQ ID NO 3 | >2500 | + |
| SEQ ID NO 4 | 0 | - |
| SEQ ID NO 5 | 0 | - |
| SEQ ID NO 6 | 0 | - |
| SEQ ID NO 7 | 773 | + |
| SEQ ID NO 8 | 560 | + |

**Tableau 25 : Barrette R2**

| Barrette R2bis | DO x 1000 | + / - |
|---|---|---|
| SEQ ID NO 9 | ND | ? |
| SEQ ID NO 10 | ND | ? |
| SEQ ID NO 11 | 0 | - |
| SEQ ID NO 12 | 0 | - |
| SEQ ID NO 13 | 0 | - |
| SEQ ID NO 14 | 0 | - |
| SEQ ID NO 15 | 0 | - |
| SEQ ID NO 16 | 0 | - |

On voit donc bien sur cette seconde analyse qu'il s'agit de DRB1*gr0405 (SEQ ID NO 7 et SEQ ID NO 8 sont positives), alors que s'il s'agissait de DRB1*gr0404 la SEQ ID NO 8 aurait dû être négative. La différenciation entre DRB1*gr0404 et DRB1*gr0405 est donc possible, ce qui a moins d'incidence que dans l'exemple précédent puisque, selon nos connaissances actuelles, ces deux allèles ont le même impact sur la polyarthrite rhumatoïde. Ainsi, DRB1*gr0404 et DRB1*gr0405 sont tous les deux associés à la susceptibilité génétique à la polyarthrite rhumatoïde. Bien entendu, l'importance de cette seconde analyse pourra varier si nos connaissances futures prouvent un impact plus important d'un de ces deux allèles par rapport à l'autre.

Le typage de ce deuxième échantillon est donc DRB1*gr0405 / gr52.

### 3°) Troisième échantillon :

**Tableau 26 : Barrette R1**

| Barrette R1 | DO x 1000 | + / - |
|---|---|---|
| SEQ ID NO 1 | >2500 | + |
| SEQ ID NO 2 | < 50 | - |
| SEQ ID NO 3 | 237 | + |
| SEQ ID NO 4 | 1 | - |
| SEQ ID NO 5 | 0 | - |
| SEQ ID NO 6 | 0 | - |
| SEQ ID NO 7 | 156 | + |
| SEQ ID NO 8 | 563 | + |

**Tableau 27 : Barrette R2**

| Barrette R2bis | DO x 1000 | + / - |
|---|---|---|
| SEQ ID NO 9 | >2500 | + |
| SEQ ID NO 10 | 637 | + |
| SEQ ID NO 11 | 2 | - |
| SEQ ID NO 12 | 4 | - |
| SEQ ID NO 13 | 0 | - |
| SEQ ID NO 14 | 0 | - |
| SEQ ID NO 15 | 0 | - |
| SEQ ID NO 16 | 248 | + |

Dans ce cas, il n'est pas possible, sans prendre de risque de différencier dans le groupe DRB1*gr04 (SEQ ID NO 3 est positive) entre DRB1*gr0404 (SEQ ID NO 7 est positive) et DRB1*gr0405 (SEQ ID NO 7 et SEQ ID NO 8 sont toutes les deux positives). L'autre allèle est un gr8+12 (SEQ ID NO 16 est positive), qui est neutre pour la polyarthrite rhumatoïde ou la spondylarthrite ankylosante, selon nos connaissances actuelles. Il faut noter également que, contrairement aux deux exemples précédents, un allèle B*27 est présent.La seconde analyse va encore une fois permettre de différencier ces deux allèles. C'est ce qui est bien représenté sur les tableaux 28 et 29.

**Tableau 28 : Barrette R1**

| Barrette R1 | DO x 1000 | + / - |
|---|---|---|
| SEQ ID NO 1 | >2500 | + |
| SEQ ID NO 2 | <50 | - |
| SEQ ID NO 3 | 1453 | + |
| SEQ ID NO 4 | 0 | - |
| SEQ ID NO 5 | 0 | - |
| SEQ ID NO 6 | 0 | - |
| SEQ ID NO 7 | 467 | + |
| SEQ ID NO 8 | 1 | - |

**Tableau 29 : Barrette R2**

| Barrette R2bis | DO x 1000 | + / - |
|---|---|---|
| SEQ ID NO 9 | ND | ? |
| SEQ ID NO 10 | ND | ? |
| SEQ ID NO 11 | 0 | - |
| SEQ ID NO 12 | 0 | - |
| SEQ ID NO 13 | 0 | - |
| SEQ ID NO 14 | 0 | - |
| SEQ ID NO 15 | 0 | - |
| SEQ ID NO 16 | 0 | - |

On voit donc bien sur cette seconde analyse qu'il s'agit de DRB1*gr0404 puisque SEQ ID NO 7 est positive et SEQ ID NO 8 est négative, alors que s'il s'agissait de DRB1*gr0405 la SEQ ID NO 8 aurait dû être positive, comme dans l'exemple précédent. La différenciation entre DRB1*gr0404 et DRB1*gr0405 est donc possible, ce qui a moins d'incidence que dans le premier exemple puisque ces deux allèles ont le même impact sur la polyarthrite rhumatoïde. Ainsi, DRB1*gr0404 et DRB1*gr0405 sont tous les deux associés à la susceptibilité génétique à la polyarthrite rhumatoïde. Néanmoins, on peut réitérer les remarques faites pour le deuxième échantillon.

Le typage de ce troisième échantillon est donc DRB1*gr0404 / gr8+12, avec présence d'au moins un allèle B*27.

### V - Conclusions :

Comme le prouve ces exemples, le procédé revendiqué permet de pratiquer simultanément, en une ou deux étapes, la recherche de susceptibilité génétique à la polyarthrite rhumatoïde, basée sur une analyse totalement dédiée aux informations attendues par le clinicien (DR1, DR4, DR10, sous-types du DRB1*gr04, présence du motif QKRAA, QRRAA et RRRAA, effet dose), et également la recherche du HLA-B*27 souvent informative lors d'une consultation en rhumatologie.

La pertinence de l'information HLA-DR et HLA-B27 est demandée lors d'une consultation en rhumatologie. Simplicité, praticabilité et rapidité du test, avec en corollaire un impact financier important, sont les avantages du procédé selon l'invention.

### Références bibliographiques

Baarsma, 1992 :
   Baarsma GS, Current Eye Researc, 1992, 11 suppl., 1-9
Benjamin, 1990 :
   Benjamin R, Parham P, Immunology Today, 1990, 11, 137-142, Guilt by association : HLA-B27 and ankylosing spondylitis
Brewerton, 1973 :
   Brewerton DA, Caffrey M, Hart FD et al, Lancet, 1973, 1, 904-907. Ankylosing spondylitis and HL-A27.
Dominguez, 1992 :
   Dominguez O, Coto E, Martinez-Naves E, Choo SY, Lopez-Larrea C, Immunogenetics, 1992, 36, 277-282. Molecular typing of HLA-B27 alleles (RM H # 311)
Gregersen, 1986 :
   Gregersen PK, Shen M, Song QL, Merryman P, Degar S, Seki T, Maccari J, Goldberg D, Murphy H, Schwenzer J, Wang CY, Winchester RJ, Nepom GT, Silver J, Proc. Natl. Acad . Sci. USA, 1986, 83, 2642-2646. Molecular diversity of HLA-DR4 haplotypes.
Gregersen, 1987 :
   Gregersen PK, Silver J, Winchester RJ, Arthritis Rheum., 1987, 30, 1205-1213. The shared epitope hypothesis. An approach to understanding the molecular genetics of susceptibility to rheumatoid arthritis.
Hill, 1991 :
   Hill AVS et al, The Lancet, 1991, March 16, 337, 640-642)
Hiraiwa, 1990 :
   Hiraiwa A, Yamanaka K, Kwok WW, Mickelson EM, Masewicz S, Hansen JA, Radka SF, Nepom GT, Proc. Natl. Acad. Sci. USA, 1990, 87, 8051-8055. Structural requirements for recognition of the HLA-Dw14 class II epitope : a key HLA determinant associated with rheumatoid arthritis.
Khan, 1979 :
   Khan MA, Kushner I, Ballou SP et al, Lancet, 1979,1, 921-922, Familial rheumatoid arthritis and HLA-DRW4.
Kirveskari, 1997 :
   Kirveskari J, Kellner H, Wuorela M, Soini H, Frankenberger B, Leirisalo-Repo M, Weiss EH, Granfors K, Br. J. Rheumatol., 1997, 36, 185-189, False-negative serological HLA-B27 typing results may be due to altered antigenic epitopes and can be detected by polymerase chain reaction.
Lawrence, 1970 :
   Lawrence J, Ann. Rheum. Dis., 1970, 29, 357-379
Nepom, 1989 :
   Nepom GT, Byers P, Seyfried C, Healey LA, Wilske KR, Stage D, Nepom BS, Arthritis Rheum. 1989, 32, 15-21. HLA genes associated with rheumatoid arthritis. Identification of susceptibility alleles using specific oligonucleotide probes.
Nepom, 1991 :
   Nepom GT, Erlich H, Annu. Rev. Immunol., 1991, 9, 493-525. MHC class-II molecules and autoimmunity.
Neumüller, 1993 :
   Neumüller J, Fischer M, Eberl R, Rheumatol. Int., 1993, 13, 163-167. Failure of the serological determination of HLA-B27 due to antigen masking in patients with ankylosing spondylitis
Schlosstein, 1973 :
   Schlosstein L, Terasaki PI, Bluestone R, Pearson CM, N. Engl. J. Med., 1973, 288, 704-706. High association of a HL-A antigen, w27, with ankylosing spondylitis
Stastny, 1978 :
   Stastny P, N. Engl. J. Med., 1978, 298, 869-871. Association of the B-cell alloantigen DRw4 with rheumatoid arthritis.
Stastny, 1983 :
   Stastny P, Ball EJ, Dry PJ, Nunez G, Immunol. Rev., 1983, 70, 113-154. The human immune response region (HLA-D) and disease susceptibility.
Stastny, 1988 :
   Stastny P, Ball E, Kahn M, Olsen N, Pincus T, Gao X, Br. J. Rheumatol., 1988, 27, 132-138. HLA-DR4 and other genetic markers in rheumatoid arthritis.
Todd, 1988 :
   Todd JA, Acha-Orbea H, Bell JI, Chao N, Fronek Z, Jacob CO, McDermott M, Sinha AA, Timmerman L, McDevitt HO, Science, 1988, 240, 1003-1009
Weiss, 1985 :
   Weiss EH, Kuon W, Doemer C, Lang M, Riethmüller G, Immunobiology, 1985, 170, 367-380, A molecular approach to analyze HLA and disease associations
Wordsworth, 1989 :
   Wordsworth BP, Lanchbury JS, Sakkas LI, Welsh KI, Panayi GS, Bell JI, Proc. Natl. Acad. Sci. USA, 1989, 86, 10049-10053

## Revendications

1. Procédé d'analyse de la prédisposition génétique d'un patient à la polyarthrite rhumatoïde, selon lequel :
on met un échantillon liquide contenant au moins un type d'amplicons, issus de l'amplification d'au moins une région polymorphe d'intérêt en rapport avec la polyarthrite rhumatoïde, en présence de sondes choisies de la façon suivante:
- au moins une sonde à faible résolution capable de s'hybrider sur le groupe d'allèles DRB1*gr04,
- au moins une sonde à haute résolution, associée à la susceptibilité génétique à la Polyarthrite Rhumatoïde, capable de s'hybrider avec les allèles suivants DRB1*0101, DRH1*gr0401, DRB1*gr0404, DRH1*gr0405, DRB1*gr0408 et DRB1*1402,
- au moins une sonde à haute résolution, associée à la résistance génétique à la Polyarthrite Rhumatoïde, capable de s'hybrider avec les allèles suivants : DRB1*gr0402, DRB1*gr0403, DRB1*gr0406 et DRB1*gr0407,
la ou les sondes à haute résolution permettant de discriminer le ou les allèles associés à la susceptibilité à la Polyarthrite rhumatoïde, et/ou le ou les allèles associés à la résistance à la Polyarthrite rhumatoïde, selon leur hybridation ou leur non hybridation.

2. Procédé, selon la revendication 1, **caractérisé en ce que** l'on utilise au moins une sonde à faible résolution capable de s'hybrider sur le groupe d'allèles DRB1*01 et sur l'allèle DRB1*10.

3. Procédé, selon la revendication 1 ou 2, afin de détecter la présence d'un autre allèle lorsqu'un seul allèle du groupe d'allèles DRB1*gr04 a été détecté, **caractérisé en ce que** l'on utilise au moins une sonde capable de s'hybrider sur les allèles suivants: DRB1*02, DRB1*03, DRB1*07, DRB1*08, DRB1*09, DRB1*11, DRB1*12, DRB1*13 et DRB1*14.

4. Procédé, selon l'une quelconque des revendications 1 à 3, **caractérisé en ce que** l'on utilise
- une sonde SEQ ID NO 3 pour le typage de DRB1*gr04,
- deux sondes à haute résolution, associées à la susceptibilité génétique à la Polyarthrite Rhumatoïde:
o SEQ ID NO 4 pour DRB1*gr0401,
o SEQ ID NO 7 pour DRB1*0101, DRB1*gr0404, DRB1*gr0405, et DRB1*gr0408, DRB1*1402,
- deux sondes à haute résolution, associées à la résistance génétique à la Polyarthrite Rhumatoïde:
o SEQ ID NO 5 pour DRB1*gr0402, et
o SEQ ID NO 6 pour DRB1*gr0403, DRB1*gr0406, DRB1*gr0407

5. Procédé, selon la revendication 4, **caractérisé en ce que** l'on utilise également une sonde à haute résolution, SEQ ID NO 8 spécifique de DRB1*gr0405, associée à la susceptibilité génétique la Polyarthrite Rhumatoïde.

6. Procédé, selon la revendication 2, **caractérisé en ce que** l'on utilise les deux sondes suivantes pour le typage:
- SEQ ID NO 11 pour le groupe d'allèles DRB1*01 et
- SEQ ID NO 15 pour l'allèle DRB1*10.

7. Procédé, selon la revendication 2, afin de détecter la présence d'un autre allèle lorsqu'un seul allèle du groupe d'allèles DRB1 a été détecté, **caractérisé en ce que** l'on utilise quatre sondes de typage suivantes :
- SEQ ID NO 13 pour DRB1*02,
- SEQ ID NO 14 pour DRB1*07, DRB1*09,
- SEQ ID NO 16 pour DRB1*08, DRB1*12,et
- SEQ ID NO 12 pour DRH1*03, DRB1*11, DRB1*13, DRB1*14.

8. Procédé, selon l'une quelconque des revendications 1 à 7, **caractérisé en ce que** chaque sonde à faible ou à haute résolution, spécifique des allèles de susceptibilité génétique à la Polyarthrite Rhumatoïde, comporte l'un des motifs caractéristiques suivants: QKRAA, QRRAA ou RRRAA.

9. Procédé, selon l'une quelconque des revendications 1 à 8, **caractérisé en ce que** moins une sonde de contrôle, capable de s'hybrider avec l'ensemble des gènes DRB1, est utilisée pour permettre la détection de tous les gènes DRB1, tel que SEQ ID NO 1 : TTC GAC AGC GAC GTG GGG.

10. Procédé, selon l'une quelconque des revendications 1 à 9, **caractérisé en ce qu'**on détecte en outre des allèles de susceptibilité génétique à la Spondylarthrite Ankylosante et autres maladies associées, et on utilise au moins une sonde à faible résolution, telle que SEQ ID NO 10, capable de s'hybrider sur le gène HLA-B, spécifique de groupe d'allèles HLA-B27.

11. Procédé, selon l'une quelconque des revendications 1 à 10, **caractérisé en ce qu'**on détecte en outre des allèles de la susceptibilité génétique associée au Lupus Erythémateux Disséminé, aux Connectivites, au Syndrome de Sjögren et autres maladies associées, **caractérisé en ce que** l'on utilise au moins une sonde à faible résolution, telle que SEQ ID NO 19, capable de s'hybrider sur le gène HLA-DR, spécifique du groupe d'allèles DRB1*03.

12. Procédé, selon l'une quelconque des revendications 1 à 11, **caractérisé en ce qu'**au plus 38,89 % des bases d'une même sonde à faible résolution ou à haute résolution est remplacée par au moins une base analogue, telle que l'inosine.

13. Procédé, selon l'une quelconque des revendications 1 à 12, **caractérisé en ce que** chaque sonde spécifique à faible résolution ou à haute résolution, est placée dans un puits d'un plaque de microtitration indépendamment des autres sondes.

14. Procédé, selon l'une quelconque des revendications 1 à 13, **caractérisé en ce que** toutes les réactions sont réalisées simultanément.

15. Procédé, selon l'une quelconque des revendications 1 à 14, **caractérisé en ce que,** préalablement, au moins une amplification de la ou des régions polymorphes d'intérêt est effectuée.

16. Procédé, selon l'une quelconque des revendications 1 à 15, **caractérisé en ce** préalablement, une amplification de la ou des régions polymorphes d'intérêt associées au HLA-DR et une amplification de la ou des régions polymorphes d'intérêt associées au HLA-B sont effectuées simultanément.

17. Procédé, selon l'une quelconque des revendications 1 à 16, **caractérisé en ce qu'**on effectue une seconde amplification d'au moins une région plus ciblée que celle déjà effectuée sur la région polymorphe, cette région plus ciblée étant une région d'intérêt en rapport avec la ou les maladies recherchées, et qu'on met les amplicons obtenus en présence des sondes précédemment définies.

18. Procédé, selon la revendication 17, **caractérisé en ce que** la première amplification est effectuée en même temps que ou préalablement à la seconde amplification plus ciblée.

19. Procédé selon la revendication 17 ou 18, **caractérisé en ce qu'**on effectue une seconde amplification d'au moins une région plus ciblée, incluse dans le groupe d'allèles DBR1*gr04, en utilisant l'amorce SEQ ID NO 27 en combinaison avec l'amorce SEQ ID NO 21.

20. Procédé selon la revendication 10, et éventuellement l'une quelconque des revendications 11 à 19**, caractérisé en ce qu'**on amplifie une séquence correspondant de l'allèle B27 en utilisant des amorces SEQ ID NO 22, SEQ ID NO 23 et/ou SEQ ID NO 25 en combinaison avec des amorces SEQ ID NO 24 et/ou SEQ ID NO 26.

21. Procédé selon la revendication 20, **caractérisé en ce qu'**on amplifie :
- une séquence correspondant aux groupes d'allèles DR en utilisant l'amorce SEQ ID NO 20 en combinaison avec l'amorce SEQ ID NO 21 et
- une séquence correspondant de l'allèle B27 en utilisant des amorces SEQ ID NO 22, SEQ ID NO 23 et/ou SEQ ID NO 25 en combinaison avec des amorces SEQ ID NO 24 et/ou SEQ ID NO 26.

22. Procédé selon la revendication 21, dans lequel la concentration finale en amorces SEQ ID NO 20 et SEQ ID NO 21, permettant l'amplification de la séquence correspondant aux groupes d'allèles DR est supérieure à la concentration finale en amorces SEQ ID NO 22, SEQ ID NO 23 et/ou SEQ ID NO 25 en combinaison avec les amorces SEQ ID NO 24 et/ou SEQ ID NO 26, permettant l'amplification de la séquence correspondant de l'allèle B27.

## Claims

1. Method for analysing a patient's genetic predisposition to rheumatoid polyarthritis, in which a liquid sample containing at least one type of amplicon, derived from the amplification of at least one polymorphic region of interest with respect to rheumatoid arthritis, in the presence of probes chosen in the following way:
- at least one low-resolution probe capable of hybridizing on the DRB1*gr04 group of alleles,
- at least one high-resolution probe associated with genetic susceptibility to rheumatoid arthritis, capable of hybridizing with the following alleles: DRB1*0101, DRB1*gr0401, DRB1*gr0404, DRB1*gr0405, DRB1*gr0408 and DRB1*gr1402,
- at least one high-resolution probe associated with genetic resistance to rheumatoid arthritis, capable of hybridizing with the following alleles: DRB1*gr0402, DRB1*gr0403, DRB1*gr0406 and DRB1*gr0407,
the high-resolution probe(s) making it possible to distinguish the allele(s) associated with susceptibility, and/or the allele(s) associated with resistance, to rheumatoid arthritis, according to whether or not they hybridize.

2. Method according to Claim 1, **characterized in that** use is made of at least one low-resolution probe capable of hybridizing on the DRB1*01 group of alleles and on the DRB1*10 allele.

3. Method according to either of Claims 1 and 2, in order to detect the presence of another allele, when a single allele of the DRB1*gr04 group of alleles has been detected, **characterized in that** use is made of at least one probe capable of hybridizing on the following alleles: DRB1*02, DRB1*03, DRB1*07, DRB1*08, DRB1*09, DRB1*11, DRB1*12, DRB1*13 and DRB1*14.

4. Method according to any one of Claims 1 to 3, **characterized in that** use is made of:
- a SEQ ID NO 3 probe, for the DRB1*gr04 typing,
- two high-resolution probes associated with genetic susceptibility to rheumatoid arthritis:
○ SEQ ID NO 4 for DRB1*gr0401,
○ SEQ ID NO 7 for DRB1*0101, DRB1*gr0404, DRB1*gr0405, DRB1*04gr08 and DRB1*1402,
- two high-resolution probes associated with genetic resistance to rheumatoid arthritis:
○ SEQ ID NO 5 for DRB1*gr0402, and
○ SEQ ID NO 6 for DRB1*gr0403, DRB1*gr0406 and DRB1*gr0407.

5. Method according to Claim 4, **characterized in that** use is also made of a high-resolution probe, SEQ ID NO 8, specific for DRB1*gr0405 and associated with genetic susceptibility to rheumatoid arthritis.

6. Method according to Claim 2, **characterized in that** the following two probes are used for the typing:
- SEQ ID NO 11 for the DRB1*01 group of alleles, and
- SEQ ID NO 15 for the DRB1*10 allele.

7. Method according to Claim 2, in order to detect the presence of another allele, when a single allele of the DRB1*gr04 group of alleles has been detected, **characterized in that** use is made of the following four typing probes:
- SEQ ID NO 13 for DRB1*02,
- SEQ ID NO 14 for DRB1*07 and DRB1*09,
- SEQ ID NO 16 for DRB1*08 and DRB1*12, and
- SEQ ID NO 12 for DRB1*03, DRB1*11, DRB1*13 and DRB1*14.

8. Method according to any one of Claims 1 to 7, **characterized in that** each low- or high-resolution probe specific for the alleles for genetic susceptibility to rheumatoid arthritis comprises one of the following characteristic motifs: QKRAA, QRRAA or RRRAA.

9. Method according to any one of Claims 1 to 8, **characterized in that** at least one control probe capable of hybridizing with the set of DRB1 genes is used to allow detection of all the DRB1 genes, such as SEQ ID NO 1: TTC GAC AGC GAC GTG GGG.

10. Method according to any one of Claims 1 to 9, **characterized in that** alleles for genetic susceptibility to ankylosing spondylitis and other associated diseases are further detected, and that use is made of at least one low-resolution probe, such as SEQ ID NO 10, capable of hybridizing on the HLA-B gene and specific for the HLA-B27 group of alleles.

11. Method according to any one of Claims 1 to 10, **characterized in that** alleles for genetic susceptibility associated with lupus erythematosus disseminatus, with collagen diseases, with Sjögren's syndrome and other associated diseases are further detected, and that use is made of at least one low-resolution probe, such as SEQ ID NO 19, capable of hybridizing on the HLA-DR gene and specific for the DRB1*03 group of alleles.

12. Method according to any one of Claims 1 to 11, **characterized in that** a maximum of 38.89% of the bases of the same low-resolution or high-resolution probe are replaced with at least one analogous base, such as inosine.

13. Method according to any one of Claims 1 to 12, **characterized in that** each specific low-resolution or high-resolution probe is placed in a well of a microtitration plate, independently of the other probes.

14. Method according to any one of Claims 1 to 13, **characterized in that** all the reactions are carried out simultaneously.

15. Method according to any one of Claims 1 to 14, **characterized in that** at least one amplification of the polymorphic region(s) of interest is carried out beforehand.

16. Method according to any one of Claims 1 to 15, **characterized in that** amplification of the polymorphic region(s) of interest associated with HLA-DR and amplification of the polymorphic region(s) of interest associated with HLA-B are carried out simultaneously, beforehand.

17. Method according to any one of Claims 1 to 16, **characterized in that** a second amplification of at least one region is carried out, which is more highly targeted than that already carried out on the polymorphic region, this more highly targeted region being a region of interest with respect to the disease(s) being sought, and **in that** the amplicons obtained are placed in the presence of the previously defined probes.

18. Method according to Claim 17, **characterized in that** the first amplification is carried out at the same time as, or prior to, the second, more highly targeted amplification.

19. Method according to Claim 17 or 18, **characterized in that** a second amplification of at least one region is carried out, which is more highly targeted, included in the DRB1*gr04 groups of alleles, in using SEQ ID NO 27 primers in combination with SEQ ID NO 21 primer.

20. Method according to Claim 10, and optionally according to any one of Claims 11 to 19, **characterized in that** an amplification of a sequence corresponding to the B27 allele, in using SEQ ID NO 22, SEQ ID NO 23 and/or SEQ ID NO 25 primers in combination with SEQ ID NO 24 and/or SEQ ID NO 26 primers is carried out.

21. Method according to Claim 20, **characterized in that** amplification is carried out of:
- a sequence corresponding to the DR groups of alleles, in using SEQ ID NO: 20 primer in combination with SEQ ID NO: 21 primer, and
- a sequence corresponding to the B27 allele, in using SEQ ID NO: 22, SEQ ID NO: 23 and/or SEQ ID NO: 25 primers in combination with SEQ ID NO: 24 and/or SEQ ID NO: 26 primers.

22. Method according to Claim 21, in which the final concentration of SEQ ID NO: 20 and SEQ ID NO: 21 primers, for amplifying the sequence corresponding to the DR groups of alleles, is higher than the final concentration of SEQ ID NO: 22, SEQ ID NO: 23 and/or SEQ ID NO: 25 primers in combination with the SEQ ID NO: 24 and/or SEQ ID NO: 26 primers, for amplifying the sequence corresponding to the B27 allele.

## Patentansprüche

1. Verfahren zur Analyse der genetische Prädisposition eines Patienten für rheumatoide Polyarthritis, in dem das Einsetzen einer flüssigen Probe, die mindestens einen Typ von Amplikons enthält, die aus der Amplifikation mindestens einer polymorphen Region von Interesse im Hinblick auf rheumatoide Polyarthritis hervorgehen, in Gegenwart von Sonden, die auf folgende Weise ausgewählt werden:
- mindestens eine Sonde mit schwacher Auflösung, die mit der Gruppe der DRB1*gr04-Allele hybridisieren kann,
- mindestens eine Sonde mit hoher Auflösung, die mit der genetischen Empfänglichkeit für rheumatoide Polyarthritis zusammenhängt und die mit den folgenden Allelen hybridisieren kann: DRB1*0101, DRB1*gr0401, DRB1*gr0404, DRB1*gr0405, DRB1*gr0408 und DRB1*1402,
- mindestens eine Sonde mit hoher Auflösung, die mit der genetischen Resistenz gegen rheumatoide Polyarthritis zusammenhängt und die mit den folgenden Allelen hybridisieren kann: DRB1*gr0402, DRB1*gr0403, DRB1*gr0406 und DRB1*gr0407,
wobei die Sonde(n) mit hoher Auflösung anhand ihrer Hybridisierung oder Nichthybridisierung eine Unterscheidung ermöglichen zwischen dem oder den Allel(en), das/die mit der Empfänglichkeit für, und/oder dem oder den Allel(en), die mit der Resistenz gegen rheumatoide Polyarthritis.

2. Verfahren nach Anspruch 1, **dadurch gekennzeichnet, dass** mindestens eine Sonde mit schwacher Auflösung verwendet wird, die mit der Gruppe der DRB1*01-Allele und dem Allel DRB1*10 hybridisieren kann.

3. Verfahren nach Anspruch 1 oder 2 zum Nachweis des Vorliegens eines anderen Allels, wenn ein einziges Allel der Gruppe der DRB1*gr04-Allele nachgewiesen wurde, **dadurch gekennzeichnet, dass** mindestens eine Sonde verwendet wird, die mit den folgenden Allelen hybridisieren kann: DRB1*02, DRB1*03, DRB1*07, DRB1*08, DRB1*09, DRB1*11, DRB1*12, DRB1*13 und DRB1*14.

4. Verfahren nach einem der Ansprüche 1 bis 3, **dadurch gekennzeichnet, dass** man verwendet:
- eine Sonde SEQ ID NR 3 zur Typisierung von DRB1*gr04,
- zwei Sonden mit hoher Auflösung, die mit der genetischen Empfänglichkeit für rheumatoide Polyarthritis zusammenhängen:
- SEQ ID NR 4 für DRB1*gr0401,
- SEQ ID NR 7 für DRB1*0101, DRB1*gr0404, DRB1*gr0405 und DRB1*04gr08, DRB1*1402,
- zwei Sonden mit hoher Auflösung, die mit der genetischen Resistenz gegen rheumatoide Polyarthritis zusammenhängen:
- SEQ ID NR 5 für DRB1*gr0402 und
- SEQ ID NR 6 für DRB1*gr0403, DRB1*gr0406, DRB1*gr0407.

5. Verfahren nach Anspruch 4, **dadurch gekennzeichnet, dass** auch eine Sonde mit hoher Auflösung, SEQ ID NR 8, verwendet wird, die für DRB1*gr0405 spezifisch ist, das mit der genetischen Empfänglichkeit für rheumatoide Polyarthritis zusammenhängt.

6. Verfahren nach Anspruch 2, **dadurch gekennzeichnet, dass** zur Typisierung die beiden folgenden Sonden verwendet werden:
- SEQ ID NR 11 für die Gruppe der DRB1*01-Allele und
- SEQ ID NR 15 für das Allel DRB1*10.

7. Verfahren nach Anspruch 2 zum Nachweis des Vorliegens eines anderen Allels, wenn ein einziges Allel der Gruppe der DRB1*gr04-Allele nachgewiesen wurde, **dadurch gekennzeichnet, dass** die folgenden vier Typisierungssonden verwendet werden:
- SEQ ID NR 13 für DRB1*02,
- SEQ ID NR 14 für DRB1*07, DRB1*09,
- SEQ ID NR 16 für DRB1*08, DRB1*12 und
- SEQ ID NR 12 für DRB1*03, DRB1*11, DRB1*13, DRB1*14.

8. Verfahren nach einem der Ansprüche 1 bis 7, **dadurch gekennzeichnet, dass** jede Sonde mit schwacher oder hoher Auflösung, die für Allele der genetischen Empfänglichkeit für rheumatoide Polyarthritis spezifisch ist, eines der folgenden charakteristischen Motive trägt: QKRAA, QRRAA oder RRRAA.

9. Verfahren nach einem der Ansprüche 1 bis 8, **dadurch gekennzeichnet, dass** mindestens eine Kontrollsonde, die mit der Gesamtheit der DRB1-Gene hybridisieren kann, verwendet wird, um den Nachweis aller DRB1-Gene zu ermöglichen, zum Beispiel SEQ ID NR 1: TTC GAC AGC GAC GTG GGG.

10. Verfahren nach einem der Ansprüche 1 bis 9, **dadurch gekennzeichnet, dass** Allelen der genetischen Empfänglichkeit für Spondylitis ankylosans und andere, damit zusammenhängende Erkrankungen darüberhinaus nachgewiesen werden, und dass mindestens eine Sonde mit schwacher Auflösung verwendet wird, wie SEQ ID NR 10, die mit dem Gen HLA-B hybridisieren kann und die für die Gruppe der HLA-B27-Allele spezifisch ist.

11. Verfahren nach einem der Ansprüche 1 bis 10, **dadurch gekennzeichnet, dass** Allelen der genetischen Empfänglichkeit in Zusammenhang mit disseminiertem Lupus erythematodes, Konnektivitiden, Sjögren-Syndrom und anderen, damit zusammenhängenden Erkrankungen, darüberhinaus nachgewiesen werden, und dass mindestens eine Sonde mit schwacher Auflösung verwendet wird, wie SEQ ID NR 19, die mit dem Gen HLA-DR hybridisieren kann und die für die Gruppe der DRB1*03-Allele spezifisch ist.

12. Verfahren nach einem der Ansprüche 1 bis 11, **dadurch gekennzeichnet, dass** höchstens 38,89% der Basen einer gleichen Sonde mit schwacher Auflösung oder mit hoher Auflösung durch mindestens eine analoge Base, wie Inosin, ersetzt wird.

13. Verfahren nach einem der Ansprüche 1 bis 12, **dadurch gekennzeichnet, dass** jede spezifische Sonde mit schwacher Auflösung oder hoher Auflösung unabhängig von den anderen Sonden in einen Brunnen einer Mikrotiterplatte eingebracht wird.

14. Verfahren nach einem der Ansprüche 1 bis 13, **dadurch gekennzeichnet, dass** alle Reaktionen gleichzeitig durchgeführt werden.

15. Verfahren nach einem der Ansprüche 1 bis 14, **dadurch gekennzeichnet, dass** zuvor mindestens eine Amplifikation der polymorphen Region(en) von Interesse durchgeführt wird.

16. Verfahren nach einem der Ansprüche 1 bis 15, **dadurch gekennzeichnet, dass** zuvor eine Amplifikation der polymorphen Region(en) von Interesse, die mit HLA-DR zusammenhäng(t/en), und eine Amplifikation der polymorphen Region(en) von Interesse, die mit HLA-B zusammenhäng(t/en), gleichzeitig durchgeführt werden.

17. Verfahren nach einem der Ansprüche 1 bis 16, **dadurch gekennzeichnet, dass** eine zweite Amplifikation mindestens einer gezielteren Region als die bereits an der polymorphen Region durchgeführte durchgeführt wird, wobei die gezieltere Region eine Region von Interesse im Hinblick auf die gesuchte(n) Erkrankung(en) ist, und dass die in Anwesenheit der vorstehend definierten Sonden erhaltenen Amplikons eingesetzt werden.

18. Verfahren nach Anspruch 17, **dadurch gekennzeichnet, dass** die erste Amplifikation gleichzeitig mit oder vor der zweiten gezielteren Amplifikation durchgeführt wird.

19. Verfahren nach Anspruch 17 oder 18, **dadurch gekennzeichnet, dass** eine zweite Amplifikation mindestens einer gezielteren Region Amplifikation durchgeführt wird, die in den Gruppen der DRB1*gr04-Allele enthalten ist, in dem man die SEQ ID NR 27-Fragmenten in Kombination mit SEQ ID NR 21-Fragmenten verwendet.

20. Verfahren nach Anspruch 19, **dadurch gekennzeichnet, dass** Amplifikation einer Sequenz, die dem Allel B27 entspricht, in dem man die SEQ ID NR 22-Fragmenten, SEQ ID NR 23-Fragmenten und/oder SEQ ID NR 25-Fragmenten in Kombination mit SEQ ID NR 24-Fragmenten und/oder SEQ ID NR 26-Fragmenten verwendet.

21. Verfahren nach Anspruch 20, **dadurch gekennzeichnet, dass** Amplifikation:
• einer Sequenz, die den Gruppen der DR-Allele entspricht, umfassend die Verwendung von SEQ ID NR 20-Fragmenten in Kombination mit SEQ ID NR 21-Fragmenten
und
• einer Sequenz, die dem Allel B27 entspricht, umfassend die Verwendung von SEQ ID NR 22-Fragmenten, SEQ ID NR 23-Fragmenten und/oder SEQ ID NR 25-Fragmenten in Kombination mit SEQ ID NR 24-Fragmenten und/oder SEQ ID NR 26-Fragmenten.

22. Verfahren nach Anspruch 21, wobei die Endkonzentration an SEQ ID NR 20-Fragmenten und SEQ ID NR 21-Fragmenten, welche die Amplifikation der Sequenz ermöglichen, die den Gruppen der DR-Allele entspricht, größer ist als die Endkonzentration an SEQ ID NR 22-Fragmenten, SEQ ID NR 23-Fragmenten und/oder SEQ ID NR 25-Fragmenten in Kombination mit den SEQ ID NR 24-Fragmenten und/oder SEQ ID NR 26-Fragmenten, welche die Amplifikation der Sequenz ermöglichen, die dem Allel B27 entspricht.


### LISTE DE SEQUENCES

<110> bioMérieux
<120> Procédé d'analyse de la prédisposition génétique d'un patient à au moins une maladie et amplification adaptée à un tel procédé
<130> HLASICK2
<140>
   <141>
<160> 27
<170> Patentln Ver. 2.1
<210> 1
   <211> 18
   <212> ADN
   <213> Homo sapiens
<400> 1
   ttcgacagcg acgtgggg 18
<210> 2
   <211> 20
   <212> ADN
   <213> Homo sapiens
<400> 2
   tatgaaactt atggggatac 20
<210> 3
   <211> 16
   <212> ADN
   <213> Homo sapiens
<400> 3
   gatacttcta tcacca 16
<210> 4
   <211> 15
   <212> ADN
   <213> Homo sapiens
<400> 4
   gagcagaanc ggncc 15
<210> 5
   <211> 15
   <212> ADN
   <213> Homo sapiens
<400> 5
   ctggaagacg ancgg 15
<210> 6
   <211> 18
   <212> ADN
   <213> Homo sapiens
<400> 6
   agcanangcn ggncnann 18
<210> 7
   <211> 18
   <212> ADN
   <213> Homo sapiens
<400> 7
   cagaggcgng nnncngtg 18
<210> 8
   <211> 15
   <212> ADN
   <213> Homo sapiens
<400> 8
   gcctagcgcc gagta 15
<210> 9
   <211> 23
   <212> ADN
   <213> Homo sapiens
<400> 9
   aaatacctca tggagtggga gcc 23
<210> 10
   <211> 18
   <212> ADN
   <213> Homo sapiens
<400> 10
   tgccttngcc ttncagac 18
<210> 11
   <211> 18
   <212> ADN
   <213> Homo sapiens
<400> 11
   tggcagctta agtttgaa 18
<210> 12
   <211> 16
   <212> ADN
   <213> Homo sapiens
<400> 12
   tactctacgt ctgagt 16
<210> 13
   <211> 17
   <212> ADN
   <213> Homo sapiens
<400> 13
   cagcctaaga gggagtg 17
<210> 14
   <211> 18
   <212> ADN
   <213> Homo sapiens
<400> 14
   naggtngaca acgtgtgc 18
<210> 15
   <211> 15
   <212> ADN
   <213> Homo sapiens
<400> 15
   ggaggaggtt aagtt 15
<210> 16
   <211> 14
   <212> ADN
   <213> Homo sapiens
<400> 16
   ctctacggn gagt 14
<210> 17
   <211> 16
   <212> ADN
   <213> Homo sapiens
<400> 17
   gaacasccag aaggac 16
<210> 18
   <211> 19
   <212> ADN
   <213> Homo sapiens
<400> 18
   ctcgctctgg ttgtagtag 19
<210> 19
   <211> 16
   <212> ADN
   <213> Homo sapiens
<400> 19
   ccgggtggac aacnac 16
<210> 20
   <211> 27
   <222> ADN
   <213> Homo sapiens
<400> 20
   ccggatcctt cgtgtcccca cagcacg 27
<210> 21
   <211> 19
   <212> ADN
   <213> Homo sapiens
<400> 21
   tcgccgctgc actgtgaag 19
<210> 22
   <211> 22
   <212> ADN
   <213> Homo sapiens
<400> 22
   gggaggagcg aggggacccc ag 22
<210> 23
   <211> 22
   <212> ADN
   <213> Homo sapiens
<400> 23
   gggaggagcg aggggaccgc ag 22
<210> 24
   <211> 18
   <212> ADN
   <213> Homo sapiens
<400> 24
   atctcggacc cggagact 18
<210> 25
   <211> 11
   <212> ADN
   <213> Homo sapiens
<400> 25
   aggggaccgc a 11
<210> 26
   <211> 20
   <212> ADN
   <213> Homo sapiens
<400> 26
   atctcggacc cggagactcg 20
<210> 27
   <211> 21
   <212> ADN
   <213> Homo sapiens
<400> 27
   gtttcttgga gcaggttaaa c 21